# EUROPEAN PATENT APPLICATION

(11) **EP 4 039 803 A1**
(43) Date of publication of application: **10.08.2022**
(21) Application number: 20871608.4
(22) Date of filing: 27.09.2020
(51) Int. Cl.: C12N 5/10, C12N 15/90, A61K 35/17, A61P 31/00, A61P 31/18, A61P 35/00

(54) **ENGINEERED HUMAN IMMUNE CELLS, PREPARATION METHOD AND APPLICATION THEREOF**

(30) Priority: 30.09.2019 CN 201910945529
(71) Applicant: Zhaotai Immugene Biomedicine (Hong Kong) Limited, Hong Kong 999077 (HK)
(72) Inventor: LI, Peng, Hong Kong 999077 (CN); JIANG, Zhiwu, Hong Kong 999077 (CN); TANG, Zhaoyang, Hong Kong 999077 (CN); QIN, Le, Hong Kong 999077 (CN); LIAO, Rui, Hong Kong 999077 (CN); ZHENG, Diwei, Hong Kong 999077 (CN); CUI, Yuanbin, Hong Kong 999077 (CN); LIN, Simiao, Hong Kong 999077 (CN); YAO, Yao, Hong Kong 999077 (CN)
(74) Representative: Cesa, Roberta
(86) International application number: PCT/CN2020/118104
(87) International publication number: WO 2021/063285

(57) **Abstract**

Provided are engineered human immune cells, a preparation method, and an application thereof. The engineered human immune cells are immune killer lymphocytes induced by reprogramming human T cells, from which the BCL11B gene is deleted. The engineered human immune cells retain markers and functions of the T cells from which they are derived and have markers and functions of NK cells. The reprogrammed engineered human immune cells can be used to prepare drugs for treating tumors and infectious diseases.

SEQUENCE LISTING

<110>  ZHAOTAI IMMUGENE BIOMEDICINE (HONG KONG) LIMITED

<120>  ENGINEERED HUMAN IMMUNE CELLS, PREPARATION METHOD AND APPLICATION
THEREOF

<130>  BY20DX0931FPPC-CN

<160>  51

<170>  PatentIn version 3.3

<210>  1
<211>  20
<212>  DNA
<213>  Homo sapiens

<400>  1
gaccctgacc tgctcacctg                                                   20


<210>  2
<211>  24
<212>  DNA
<213>  Artificial sequence

<220>
<223>  Forward primer for the second exon target sequence

<400>  2
caccgaccct gacctgctca cctg                                              24


<210>  3
<211>  24
<212>  DNA
<213>  Artificial sequence

<220>
<223>  Reverse primer for the second exon target sequence

<400>  3
aaaccaggtg agcaggtcag ggtc                                              24


<210>  4
<211>  20
<212>  DNA
<213>  Homo sapiens

<400>  4
gaagcagtgt ggcggcagct                                                   20


<210>  5
<211>  24
<212>  DNA
<213>  Artificial sequence

<220>
<223>  Forward primer for the second exon target sequence
<400>  5

caccgaagca gtgtggcggc agct                                              24


<210>  6
<211>  24
<212>  DNA
<213>  Artificial sequence

<220>
<223>  Reverse primer for the second exon target sequence

<400>  6
aaacagctgc cgccacactg cttc                                              24


<210>  7
<211>  20
<212>  DNA
<213>  Homo sapiens

<400>  7
caggtggtca tcttcgtcgg                                                   20


<210>  8
<211>  24
<212>  DNA
<213>  Artificial sequence

<220>
<223>  Forward primer for the second exon target sequence
<400>  8
cacccaggtg gtcatcttcg tcgg                                              24


<210>  9
<211>  24
<212>  DNA
<213>  Artificial sequence

<220>
<223>  Reverse primer for the second exon target sequence

<400>  9
aaacccgacg aagatgacca cctg                                              24


<210>  10
<211>  19
<212>  DNA
<213>  Homo sapiens

<400>  10
gcaggtggtc atcttcgtc                                                    19


<210>  11
<211>  23
<212>  DNA
<213>  Artificial sequence

<220>
<223>  Forward primer for the second exon target sequence

<400>  11
caccgcaggt ggtcatcttc gtc                                              23


<210>  12
<211>  23
<212>  DNA
<213>  Artificial sequence

<220>
<223>  Reverse primer for the second exon target sequence

<400>  12
aaaccgacga agatgaccac ctg                                              23


<210>  13
<211>  20
<212>  DNA
<213>  Homo sapiens

<400>  13
gctcaggaaa gtgtccgagc                                                  20


<210>  14
<211>  24
<212>  DNA
<213>  Artificial sequence

<220>
<223>  Forward primer for the second exon target sequence
<400>  14
caccgctcag gaaagtgtcc gagc                                             24


<210>  15
<211>  23
<212>  DNA
<213>  Artificial sequence

<220>
<223>  Reverse primer for the second exon target sequence

<400>  15
aaacgctcgg acactttcct gag                                              23


<210>  16
<211>  20
<212>  DNA
<213>  Homo sapiens

<400>  16
gagtcccgtc acccgagacc                                                  20


<210>  17
<211>  24
<212>  DNA
<213>  Artificial sequence

<220>

<223>  Forward primer for the third exon target sequence

<400>  17
caccgagtcc cgtcacccga gacc                                              24


<210>  18
<211>  23
<212>  DNA
<213>  Artificial sequence

<220>
<223>  Reverse primer for the third exon target sequence

<400>  18
aaacggtctc gggtgacggg act                                               23


<210>  19
<211>  20
<212>  DNA
<213>  Homo sapiens

<400>  19
gaagtgatca cggatgagtg                                                   20


<210>  20
<211>  24
<212>  DNA
<213>  Artificial sequence

<220>
<223>  Forward primer for the third exon target sequence

<400>  20
caccgaagtg atcacggatg agtg                                              24


<210>  21
<211>  23
<212>  DNA
<213>  Artificial sequence

<220>
<223>  Reverse primer for the third exon target sequence

<400>  21
aaaccactca tccgtgatca ctt                                               23


<210>  22
<211>  20
<212>  DNA
<213>  Homo sapiens

<400>  22
ggtgacggga ctcagggtga                                                   20


<210>  23
<211>  24
<212>  DNA

<213>  Artificial sequence

<220>
<223>  Forward primer for the third exon target sequence

<400>  23
caccggtgac gggactcagg gtga                                              24


<210>  24
<211>  23
<212>  DNA
<213>  Artificial sequence

<220>
<223>  Reverse primer for the third exon target sequence

<400>  24
aaactcaccc tgagtcccgt cac                                               23


<210>  25
<211>  20
<212>  DNA
<213>  Homo sapiens

<400>  25
tgcagcgcgc gcccggtctc                                                   20


<210>  26
<211>  24
<212>  DNA
<213>  Artificial sequence

<220>
<223>  Forward primer for the third exon target sequence

<400>  26
cacctgcagc gcgcgcccgg tctc                                              24


<210>  27
<211>  24
<212>  DNA
<213>  Artificial sequence

<220>
<223>  Reverse primer for the third exon target sequence

<400>  27
aaacgagacc gggcgcgcgc tgca                                              24


<210>  28
<211>  20
<212>  DNA
<213>  Homo sapiens

<400>  28
cacgagagcg acccgtcgct                                                   20

<210>  29
<211>  24
<212>  DNA
<213>  Artificial sequence

<220>
<223>  Forward primer for the fourth exon target sequence

<400>  29
cacccacgag agcgacccgt cgct                                              24


<210>  30
<211>  24
<212>  DNA
<213>  Artificial sequence

<220>
<223>  Reverse primer for the fourth exon target sequence

<400>  30
aaacagcgac gggtcgctct cgtg                                              24


<210>  31
<211>  20
<212>  DNA
<213>  Homo sapiens

<400>  31
gcgacgggtc gctctcgtgg                                                   20


<210>  32
<211>  24
<212>  DNA
<213>  Artificial sequence

<220>
<223>  Forward primer for the fourth exon target sequence

<400>  32
caccgcgacg ggtcgctctc gtgg                                              24


<210>  33
<211>  23
<212>  DNA
<213>  Artificial sequence

<220>
<223>  Reverse primer for the fourth exon target sequence

<400>  33
aaacccacga gagcgacccg tcg                                               23


<210>  34
<211>  20
<212>  DNA
<213>  Homo sapiens

<400>  34

tccatgctga agctcgactc                                                    20


<210>  35
<211>  24
<212>  DNA
<213>  Artificial sequence

<220>
<223>  Forward primer for the fourth exon target sequence

<400>  35
cacctccatg ctgaagctcg actc                                              24


<210>  36
<211>  24
<212>  DNA
<213>  Artificial sequence

<220>
<223>  Reverse primer for the fourth exon target sequence

<400>  36
aaacgagtcg agcttcagca tgga                                              24


<210>  37
<211>  20
<212>  DNA
<213>  Homo sapiens

<400>  37
acgggtcgct ctcgtggtgg                                                    20


<210>  38
<211>  24
<212>  DNA
<213>  Artificial sequence

<220>
<223>  Forward primer for the fourth exon target sequence

<400>  38
caccacgggt cgctctcgtg gtgg                                              24


<210>  39
<211>  24
<212>  DNA
<213>  Artificial sequence

<220>
<223>  Reverse primer for the fourth exon target sequence

<400>  39
aaacccacca cgagagcgac ccgt                                              24


<210>  40
<211>  20
<212>  DNA

<213>  Homo sapiens

<400>  40
agccgcaacc gcgagaacgg                                                   20


<210>  41
<211>  24
<212>  DNA
<213>  Artificial sequence

<220>
<223>  Forward primer for the fourth exon target sequence

<400>  41
caccagccgc aaccgcgaga acgg                                              24


<210>  42
<211>  24
<212>  DNA
<213>  Artificial sequence

<220>
<223>  Reverse primer for the fourth exon target sequence

<400>  42
aaacccgttc tcgcggttgc ggct                                              24


<210>  43
<211>  20
<212>  DNA
<213>  Homo sapiens

<400>  43
gcaacttgac ggtgcaccgg                                                   20


<210>  44
<211>  24
<212>  DNA
<213>  Artificial sequence

<220>
<223>  Forward primer for the fourth exon target sequence

<400>  44
caccgcaact tgacggtgca ccgg                                              24


<210>  45
<211>  23
<212>  DNA
<213>  Artificial sequence

<220>
<223>  Reverse primer for the fourth exon target sequence

<400>  45
aaacccggtg caccgtcaag ttg                                               23

<210>  46
<211>  20
<212>  DNA
<213>  Homo sapiens

<400>  46
gagctgggcc gcccggggcc                                                   20


<210>  47
<211>  24
<212>  DNA
<213>  Artificial sequence

<220>
<223>  Forward primer for the fourth exon target sequence

<400>  47
caccgagctg ggccgcccgg ggcc                                              24


<210>  48
<211>  23
<212>  DNA
<213>  Artificial sequence

<220>
<223>  Reverse primer for the fourth exon target sequence

<400>  48
aaacggcccc gggcggccca gct                                               23


<210>  49
<211>  20
<212>  DNA
<213>  Homo sapiens

<400>  49
ggtcagacgg aggctccctt                                                   20


<210>  50
<211>  24
<212>  DNA
<213>  Artificial sequence

<220>
<223>  Forward primer for the third exon target sequence

<400>  50
caccggtcag acggaggctc cctt                                              24


<210>  51
<211>  24
<212>  DNA
<213>  Artificial sequence

<220>
<223>  Reverse primer for the third exon target sequence

<400>  51

aaacaaggga gcctccgtct gacc 24

## Description

### TECHNICAL FIELD

The present application relates to the technical field of biomedicine, in particular, engineered human immune cells, a preparation method, and an application thereof.

### BACKGROUND

At present, immunotherapy has become the most concerned and promising "new" idea in the field of cancer treatment. In 2013, in the top ten scientific breakthroughs ranked by *Science*, tumor immunotherapy topped the list. Chimeric antigen receptor (CAR) T cells produced by Novartis and Kite Pharma have been approved by Food and Drug Administration (FDA), U.S., and the tumor immunotherapy has made milestone progress in the field of hematological malignancies treatment. However, there are still technical bottlenecks in the clinical treatment of tumor immunotherapy, for example, the single target of genetically modified immune cells leads to tumor immune escape and tumor recurrence; there is a lack of specific markers with high efficiency and low side effects for solid tumors, and the current gene-modified immunotherapies show no efficient and safe clinical effect on solid tumors.

T cells can be grouped into different subsets according to their surface markers and functions. For example, according to the difference in TCR types, T cells can be classified into γδ T cells and αβ T cells. αβ T cells account for more than 95% of T cells, are the main cell groups that have T cell differentiation markers and execute T cell functions *in vivo,* and represent the diversity of T cells. γδ T cells are a group of highly heterogeneous cells, whose surface T cell receptor of γδ T cells is composed of γ chain and δ chain, γδ T cells have features of various subtype, variable phenotype and rich functions, the biological characteristics of γδ T cell subtypes are different, and γδ T cells play an important role in the occurrence and development of tumors, infections and autoimmune diseases and are considered as the bridge spanning innate immunity and adaptive immunity.

Like T cells, natural killer (NK) cells are an indispensable part of the human immune system. NK cells are considered to be lymphoid cells which account for about 10% to 15% of peripheral blood lymphocytes and play a key role in the innate immune response. Different from T cells, NK cells recognize their targets in an MHC-unrestricted manner. NK cells have antiviral, anti-GvH and anti-cancer effects. Specifically, NK cells directly kill malignant tumors including sarcomas, myelomas, cancers, lymphomas and leukemia, or eliminate abnormal cells that are tumor cells or cells developing into tumor cells by inducing the activity of dendritic cells (DCs) or the adaptive immune activation of tumor-specific cytotoxic T lymphocytes (CTLs). Although NK cells have the potential to be used as therapeutic agents against cancers or infectious diseases, most NK cells in the normal human body are present in a dormant state, and NK cells in cancer patients lose their functions due to the immune escape mechanism of cancer cells. In order to use natural killer cells as therapeutic agents, activated natural killer cells that can recognize and destroy tumor cells are required. Because the number of natural killer cells *in vivo* is limited, it is very important to obtain a sufficient number of activated natural killer cells.

Cell reprogramming refers to the process that differentiated cells are reversed under specific conditions and then return to the totipotent state, or form embryonic stem cell lines, or further develop into a new individual. In the field of immunotherapy of diseases, there have been reports on the transformation of immune cells by cell reprogramming. For example, Dr. Ding Sheng from Gladstone Institutes in the United States reported that pro-inflammatory effector T cells were reprogrammed into anti-inflammatory regulatory T cells by specific reprogramming. Such reprogramming is of great significance to the treatment of autoimmune diseases. Specifically, for autoimmune diseases, over-stimulated effector T cells cause damage to the body, and when these cells are transformed into regulatory T cells, the overactivity of the immune system can be reduced and thus the immune system restores balance, thereby fundamentally treating the diseases.

At present, there is no application of cell reprogramming in human tumor immunotherapy.

### SUMMARY

In view of the deficiency of the prior art and the actual demands, the present application provides human immune cells engineered by reprogramming, a preparation method, and an application thereof. The engineered human immune cells of the present application have partial markers and functions of T cells and NK cells and simultaneously express antigen recognition and killing receptors of NK cells and T cells, and thus have a wider spectrum of tumor antigen recognition and killing functions than NK cells and T cells. Meanwhile, compared with human mature T cells from which the engineered human immune cells are derived, the engineered human immune cells of the present application have an enhanced amplification ability and a better anti-tumor effect.

In a first aspect, the present application provides immune killer lymphocytes induced by reprogramming of human T cells (induced T-to-natural killer (ITNK) cells), which retain markers and functions of T cells from which the immune killer lymphocytes are derived and have markers and functions of NK cells, wherein the reprogramming of the human T cells involves deletion of a BCL11B gene.

Preferably, the human T cells are mature human T cells or a cell population including mature human T cells; further preferably, the mature human T cells or the cell population including the mature human T cells are derived from human cord blood or human peripheral blood; and further preferably, the mature human T cells or the cell population including the mature human T cells are derived from mature T cells or cell populations obtained by differentiation of pluripotent stem cells, embryonic stem cells or cord blood stem cells.

Preferably, the reprogrammed immune killer lymphocytes express functional TCR, CD3 and NKp30.

Preferably, the reprogrammed immune killer lymphocytes express a marker of NK cells selected from the following group consisting of CD11c, NKG2D and CD161.

Preferably, immunosuppression checkpoints PD-1, CTLA-4 and FOXP3 are of low expression or no expression in the reprogrammed immune killer lymphocytes.

Preferably, NK-related markers CD127, CD16, KIRDL2, KIRDL3 and NKG2A are of low expression or no expression in the reprogrammed immune killer lymphocytes.

Preferably, compared with the T cells from which the immune killer lymphocytes are derived, expression of NOTCH on the reprogrammed immune killer lymphocytes is up-regulated.

Preferably, compared with the T cells from which the immune killer lymphocytes are derived, expression of transcription factors LEF1 and TCF7 is down-regulated, and expression of NOTCH, AP1, mTOR, ID2, TBX21 and NFIL3 is up-regulated.

Preferably, TCR-mediated signal transduction of the reprogrammed immune killer lymphocytes is enhanced.

Preferably, compared with the T cells from which the immune killer lymphocytes are derived, expression of genes of the reprogrammed immune killer lymphocytes, which are related to the TCR-mediated signal transduction and include CSF2, FOS, MAPK12, MAP3K8, IFNγ, NFKBIA, MAPK11, IL-10 and TEC, is up-regulated.

Preferably, compared with NK cells, T cell recognition and TCR signal transduction of the reprogrammed immune killer lymphocytes are enhanced; and preferably, expression of CD3, CD4, CD8 and CD40LG is up-regulated.

Preferably, compared with the T cells from which the immune killer lymphocytes are derived, NK killing toxicity-related signal transduction of the reprogrammed immune killer lymphocytes is enhanced.

Preferably, compared with the T cells from which the immune killer lymphocytes are derived, expression of genes of the reprogrammed immune killer lymphocytes, which are related to the NK killing toxicity-related signal transduction and include PRF1, CSF2, ICAM1, CD244, PLCG2, IFNG, FCER1G, GZMB, NCR2, NCR1, KIR2DL4 and SYK, is up-regulated.

In a preferred specific embodiment, the reprogrammed immune killer lymphocytes include cell subsets CD8+NKp46^{hi}NKp44+NKp30+, CD4+NKp30+, and yδTCR+NKp46^{hi}NKp44+NKp30+T.

In a preferred specific embodiment, the human T cells are mature human T cells, and the reprogramming of the human T cells includes:
1) activating mature human T cells;
2) performing BCL11B gene knockout on the activated mature human T cells obtained in step 1); and
3) culturing the cells obtained in step 2) with a T cell culture medium.

Wherein, in step 1), the activation is performed using an anti-human CD3 antibody, an anti-human CD28 antibody, and an anti-human CD2 antibody.

Preferably, magnetic beads of anti-human CD3 antibody, anti-human CD28 antibody and anti-human CD2 antibody are mixed with the mature human T cells in a ratio of 1:2 and incubated to activate the T cells.

In step 3), the T cell medium includes IL-2; and preferably, the cells obtained in step 2) are not co-cultured with OP9-DL1.

In a second aspect, the present application provides a method for preparing the cells described in the first aspect, including:
1') activating human T cells;
2') performing BCL11B gene knockout on the activated human T cells obtained in step 1'); and
3') culturing the cells obtained in step 2') with a T cell culture medium.

In the above-mentioned method, preferably, the human T cells are mature human T cells or a cell population including mature human T cells; further preferably, the mature human T cells or the cell population including the mature human T cells are derived from human cord blood or human peripheral blood; and further preferably, the mature human T cells or the cell population including the mature human T cells are derived from mature T cells or cell populations obtained by differentiation of pluripotent stem cells, embryonic stem cells or cord blood stem cells.

In the above-mentioned method, preferably, in step 1'), the activation is performed using an anti-human CD3 antibody, an anti-human CD28 antibody, and an anti-human CD2 antibody; and in a preferred specific embodiment, magnetic beads of anti-human CD3 antibody, anti-human CD28 antibody and anti-human CD2 antibody are mixed with the mature human T cells in a ratio of 1:2 and incubated to activate the T cells.

Preferably, in step 2'), the BCL11B gene knockout is performed by CRISPR/CAS9; and further preferably, the gene knockout is performed at a second exon and/or a third exon of a BCL11B gene.

Preferably, in step 3'), the T cell medium includes IL-2; and preferably, the cells obtained in step 2') are not co-cultured with OP9-DL1.

In a third aspect, the present application provides an application of the cells described in the first aspect in the preparation of a medicament for the treatment of a disease selected from the group consisting of tumors, AIDS and infectious diseases; and preferably, the infectious diseases are viral infectious diseases.

Preferably, the medicament further includes a pharmaceutically acceptable excipient.

The present application is the first to implement the reprogramming of human T cells into immune killer lymphocytes. The reprogrammed cells simultaneously express antigen recognition killer receptors of NK cells and T cells, especially functional TCRs, and have the functions of T cells and NK cells; and since the reprogrammed cells simultaneously express antigen recognition and killing receptors of NK cells and T cells, the reprogrammed cells can recognize antigens sensitive to these receptors. Therefore, compared with T cells and NK cells, the reprogrammed cells not only have a wider spectrum of tumor antigen recognition and killing functions, but also have a wider spectrum of microbial recognition and removal functions on microorganisms such as viruses and bacteria.

In addition, the reprogrammed cells of the present application have an efficient *in vitro* amplification ability. In adoptive cell transfer (ACT) therapy, both T cells and NK cells are used to treat cancers. The reprogrammed cells of the present application have the functions of both T cells and NK cells, and free from the limitation of availability and amplification ability of NK cells in the adoptive immunotherapy (ACT), the user can obtain a large number of T cells from the peripheral blood of a patient to generate the reprogrammed immune killer lymphocytes of the present application, and within 2 to 3 weeks, the user can obtain 200×10⁶ to 1248×10⁶ reprogrammed immune killer lymphocytes from about 100×10⁶ peripheral blood mononuclear cells (PBMC) of a solid tumor patient, thereby meeting the demand of the patient for cell reinfusion.

### BRIEF DESCRIPTION OF DRAWINGS

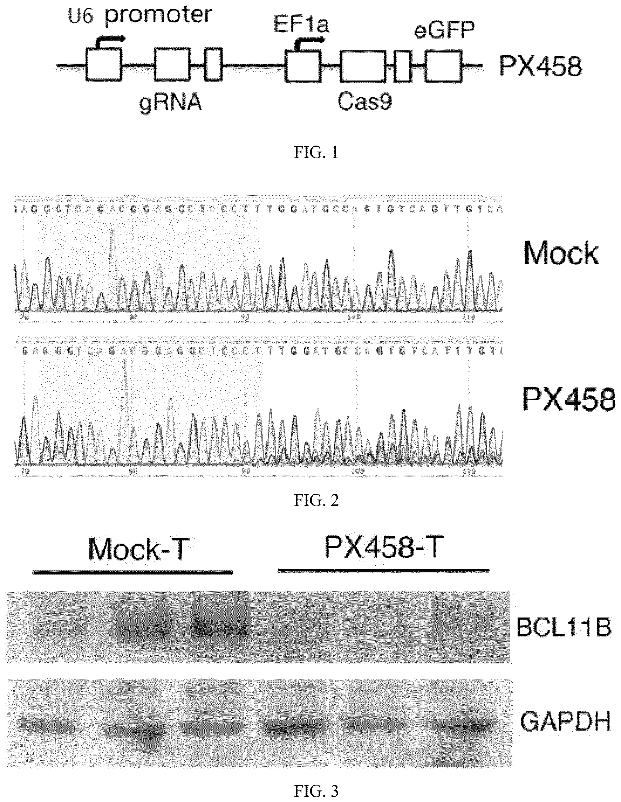

FIG. 1 is a schematic diagram of a PX458-gBCL11B vector constructed in Example 1 of the present application.
FIG. 2 is a graph showing detection and verification by gene sequencing of whether BCL11B exons of T cells transduced with PX458-gBCL11B had been knocked out, and the control group was T cells transduced with PX458 empty vectors (Mock).
FIG. 3 is a graph showing detection and verification by Western Blotting of the expression level of BCL11B proteins in T cells transduced with PX458-gBCL11B to further confirm whether BCL11B proteins were deleted, and the control group was T cells transduced with PX458 empty vectors (Mock).

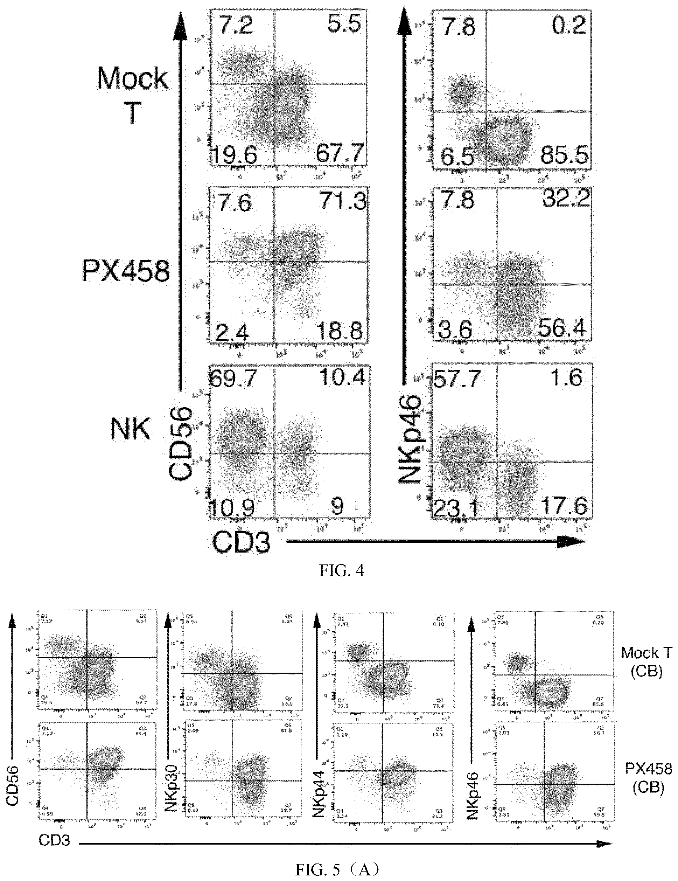

FIG. 4 is a scatter plot of flow cytometry results, showing that compared with Mock T cells and NK cells, the ITNK cells of the present application (i.e., PX458-gBCL11B-transduced T cells, indicated by PAX458 in the figure) simultaneously highly expressed the T cell marker CD3 and the NK cell markers CD56 and NKp46. All samples were derived from the same PBMC sample, among which Mock T cells were sorted from PBMC through Pan-T, so there was 7.2% to 7.8% CD3-CD56+NKp46+NK cell mixed population; PX458 cells were obtained by transfection of Mock T cells with PX458-gBCL11B, so there was 7.6% to 7.8% NK cell subset that was the same as that in Mock T cells; and NK cells were obtained by culturing and purification of PBMC through an NK cell culture medium, so there was a small amount of 10.4% NKT or γδT cell mixed population of CD3+CD56+.

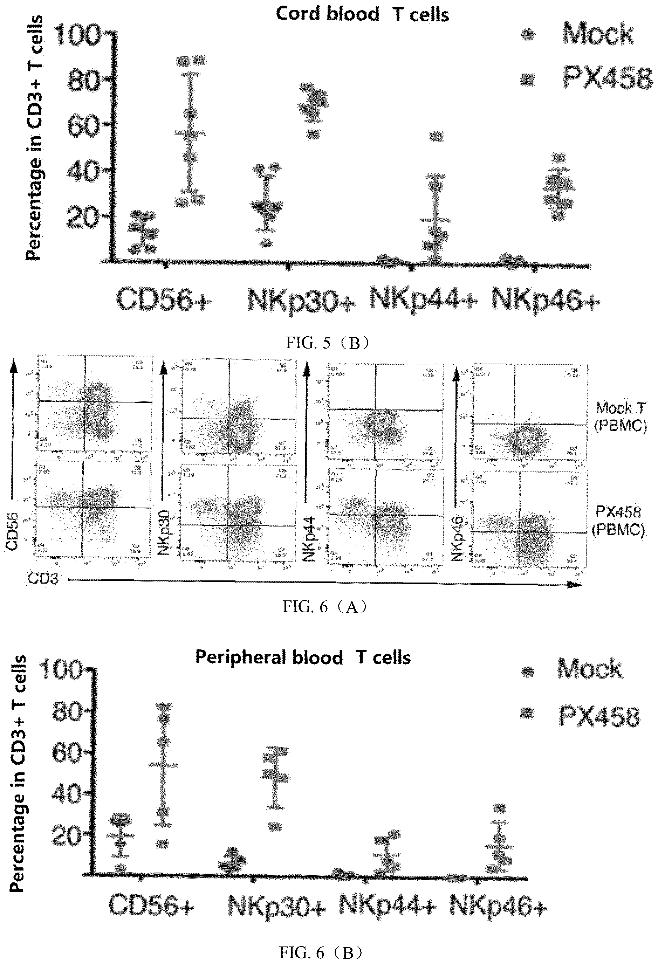

FIG. 5 is a scatter plot (A) of flow cytometry results, showing that compared with Mock T cells, T cell markers CD3 and NK cell markers NKp46, CD56, NKp30 and NKp44 were of high expression in cord blood-derived ITNK cells (i.e., PX458-gBCL11B-transduced T cells, indicated by PAX458 in the figure), and a corresponding cell percentage statistical graph (B).
FIG. 6 is a scatter plot (A) of flow cytometry results, showing that compared with Mock T cells, T cell markers CD3 and NK cell markers NKp46, CD56, NKp30 and NKp44 are of high expression in peripheral blood-derived ITNK cells (i.e., PPX458-gBCL11B-transduced T cells, indicated by PAX458 in the figure), and a corresponding cell percentage statistical graph (B).

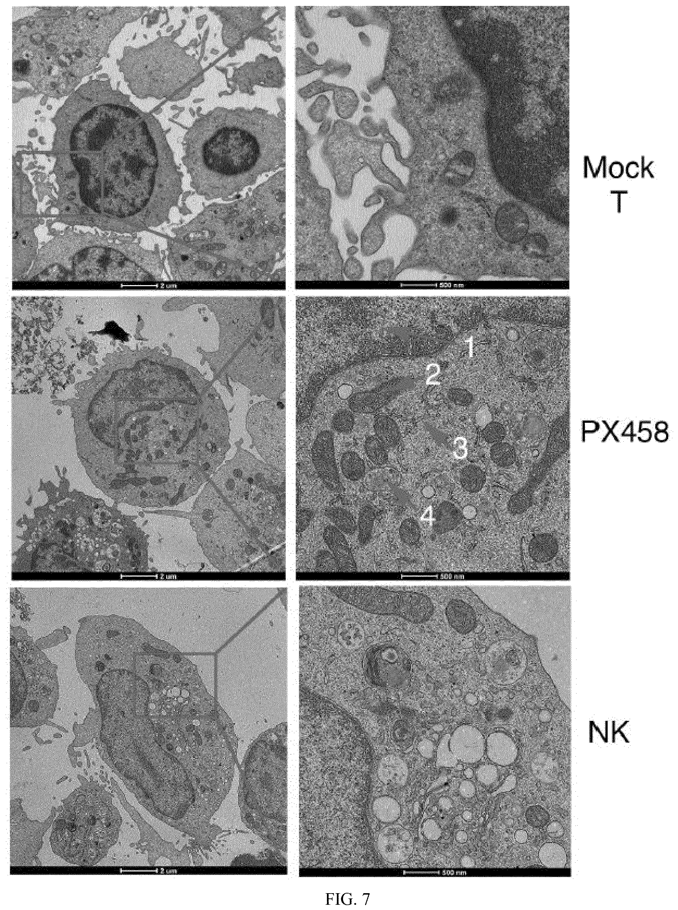

FIG. 7 is transmission electron microscopic images of Mock T cells, NK cells and ITNK cells (i.e., PX458-gBCL11B-transduced T cells, indicated by PAX458 in the figure), showing that the nucleoplasm of ITNK cells was lower than the nucleoplasm of T cells; in which 1 indicates the core, 2 indicates mitochondria, 3 indicates endoplasmic reticulum, and 4 indicates granules, with a scale of 2 µm in the left images and a scale of 500 nm in the right images.

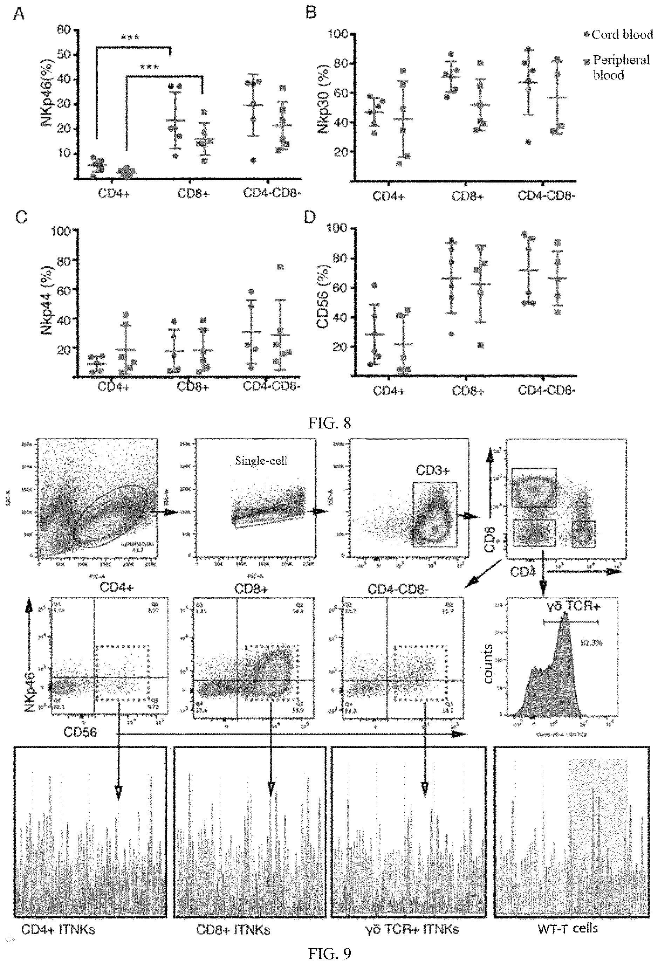

FIG. 8 shows the expression of NK receptors in T cell subsets CD4+, CD8+, CD3+CD4-CD8-of PX458-gBCL11B-transduced T cells (i.e., ITNK cells) derived from cord blood and peripheral blood, showing that the expression of NKp46 in CD8+ T cells was significantly higher than the expression of NKp46 in CD4+ T cells; in which n = 6, indicating that the experiment was a duplicate of 6 independent healthy donors, ^{∗∗∗}P ≤ 0.001, and a paired t test was adopted.
FIG. 9 shows flow cytometry analysis of PX458-gBCL11-transduced T cells, in which CD56+ cells in subsets CD4+, CD8+ and CD4-CD8- were sorted and subjected to DNA sequenced, which further confirmed that the BCL11B site in ITNK cells was knocked out.

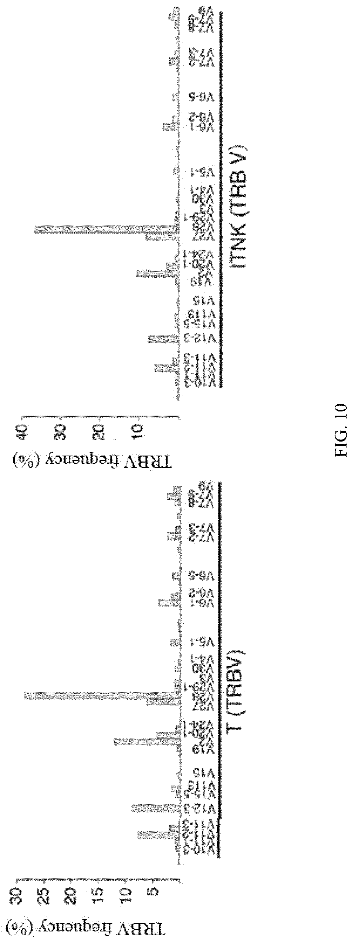

FIG. 10 shows the sequencing of TCRβ diversity, showing that all TCRβ chain sequence trajectory variables had the same diversity in the same source of T cells and ITNK cells.

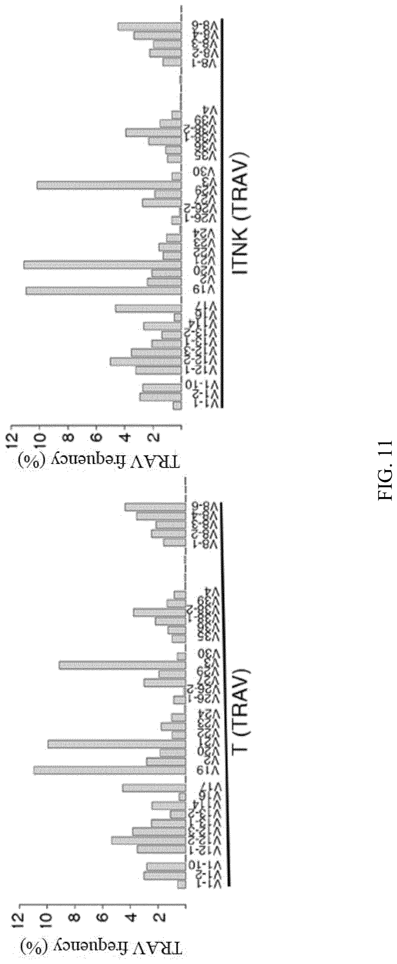

FIG. 11 shows the sequencing of TCRα diversity, showing that all TCRα chain sequence trajectory variables had the same diversity in the same source of T cells and ITNK cells.

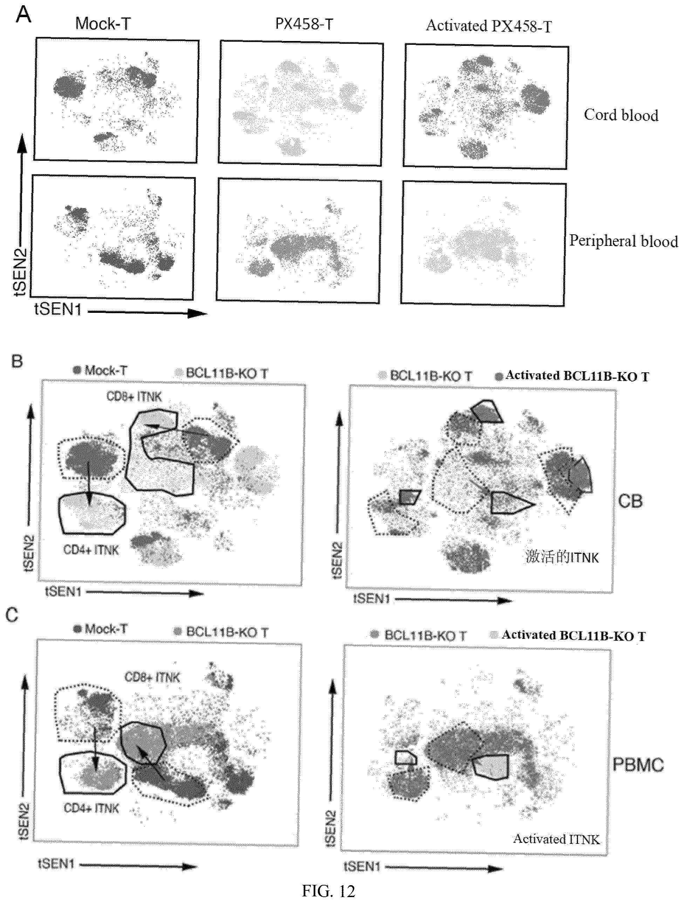

FIG. 12 is t-SEN dot density graphs of mass cytometry clustering analysis, in which (A) shows t-SEN dot densities of CD45+ monocytes derived from cord blood and peripheral blood, respectively, where CD45+ monocytes were divided into three experimental groups: cells (Mock-T) transduced with PX458 empty vectors, cells (PX458-T) transduced with PX458-gBCL11B and cells (activated PX458-T) that were transduced with PX458-gBCL11B and then activated by K562 cells for 24 hours; and (B) and (C) show clustering differences and over-transformation (indicated by arrows respectively) between cord blood (CB)-derived Mock-T cells and CB-derived T cells with BCL11B knocked out (BCL11B-KO T), CB-derived T cells with BCL11B knocked out (BCL11B-KO T) and activated CB-derived T cells with BCL11B knocked out (Activated BCL11B-KO T), adult peripheral blood (PBMC)-derived Mock-T cells and PBMC-derived T cells with BCL11B knocked out (BCL11B-KO T), PBMC-derived T cells with BCL11B knocked out (BCL11B-KO T) and activated PBMC-derived T cells with BCL11B knocked out (Activated BCL11B-KO T), respectively.

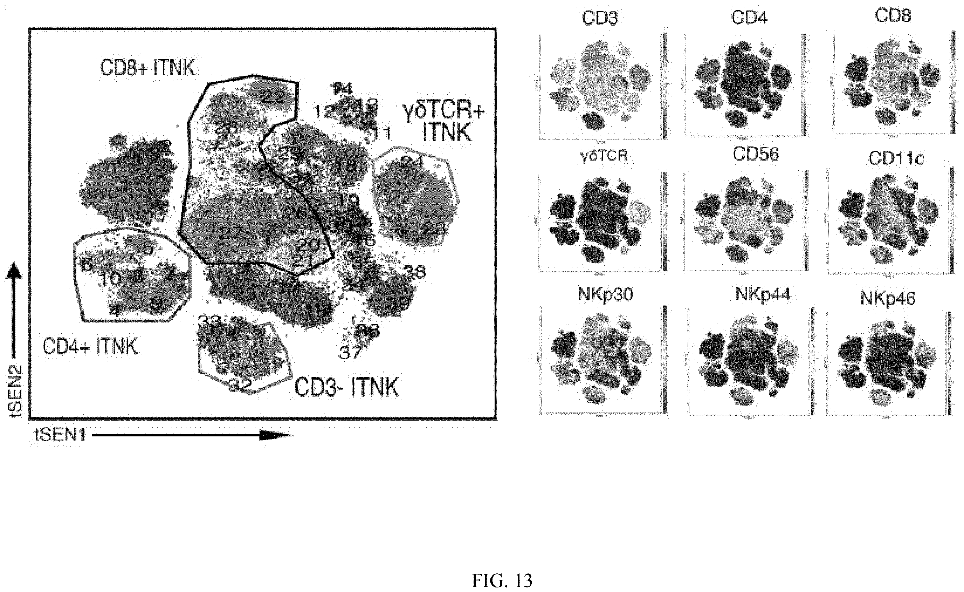

FIG. 13 is t-SEN color enrichment cluster graphs of cord blood and peripheral blood CD45+ cells fusion, in which ITNK cells are marked as indicated, where the standardized expression of CD3, CD4, CD8, yδ TCR, CD56, NKp46, NKp30, NKp44 and CD11c on t-SEN graphs stained the cells.

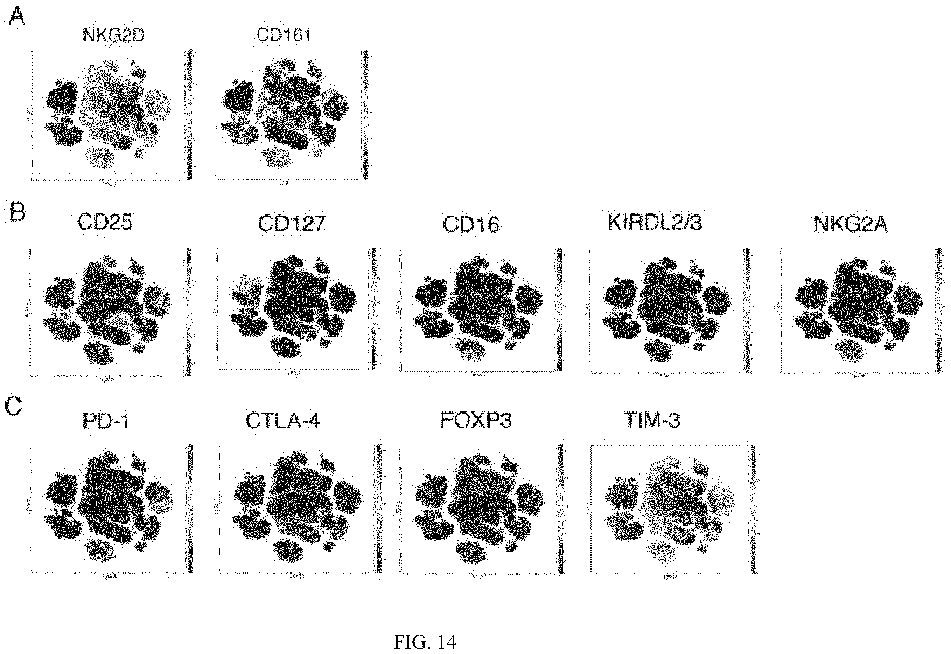

FIG. 14 is t-SEN color enrichment cluster graphs of ITNK cell immunophenotypic analysis-cord blood and peripheral blood CD45+ cells fusion; in which (A) shows the expression levels of markers NKG2D and CD161, (B) shows the expression levels of markers CD25, CD127, CD16, KIRDL2, KIRDL3 and NKG2A, and (C) shows the expression levels of immune checkpoint markers PD-1, CTLA-4, FOXP3 and TIM-3.

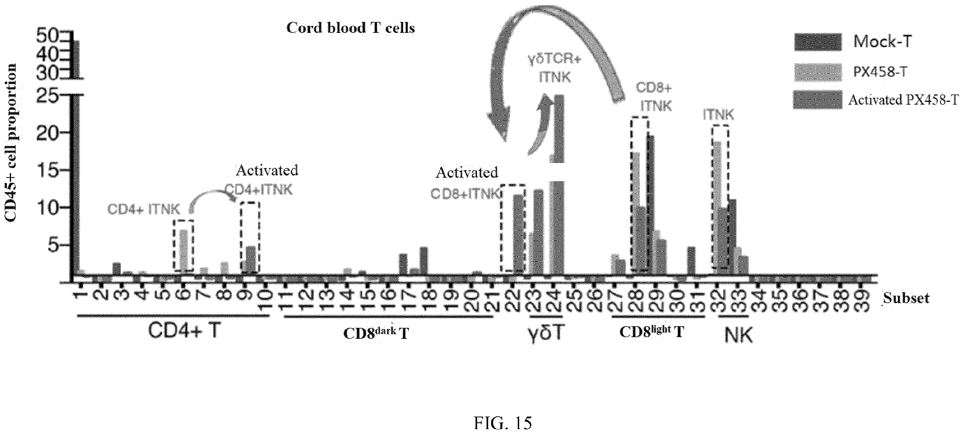

FIG. 15 shows that according to the grouping frequency of immunocyte subsets of cord blood T cells, ITNK cells can be differentiated from CD4+ T cells, CD8+ T cells and γδTCR+ T cells, and all ITNK subtypes can be activated by HLA negative cells (K562).

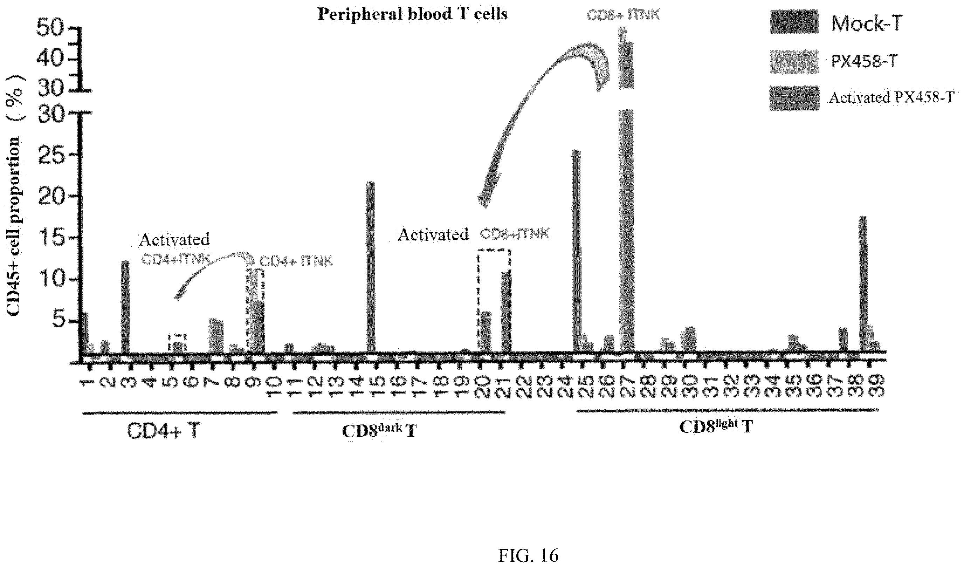

FIG. 16 shows that according to the grouping frequency of immunocyte subsets of peripheral blood T cells, ITNK cells can be differentiated from CD4+ T cells, CD8+ T cells and δγTCR+ T cells, and all ITNK subtypes can be activated by HLA negative cells (K562).

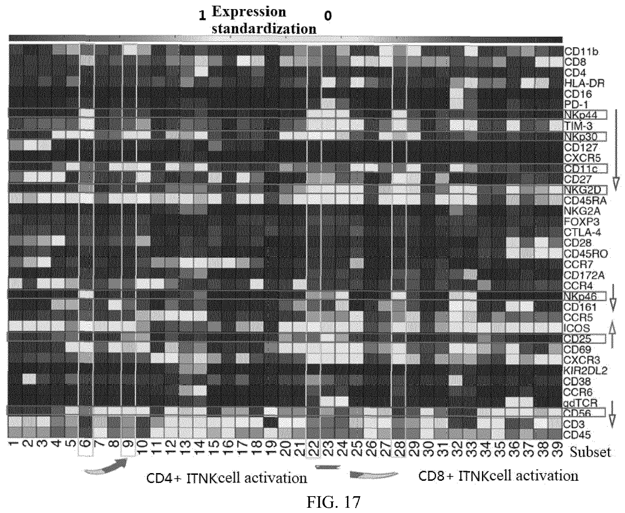

FIG. 17 shows a heatmap of mass cytometry analysis results, which shows the expression of markers for each immune cell subset.

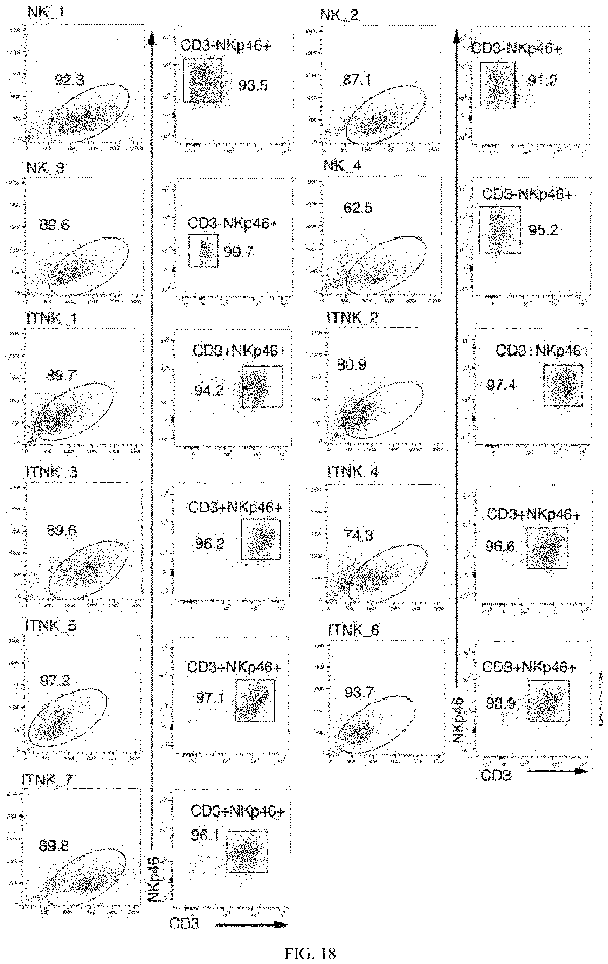

FIG. 18 shows ITNK cells sorting for RNA-Seq. CD3+ T, CD3-NKp46+ NK, CD3+NKp46+ CB-ITNK and PBMC-ITNK were sorted from cord blood or adult peripheral blood for RNA sequencing and atac sequencing. The purity of sorted cells from Mock-T cells (n=6), ITNK cells (n=6) and NK cells (n=4) was 95.3±3.61%, 92.41±2.6% and 92.88±2.06%, respectively.

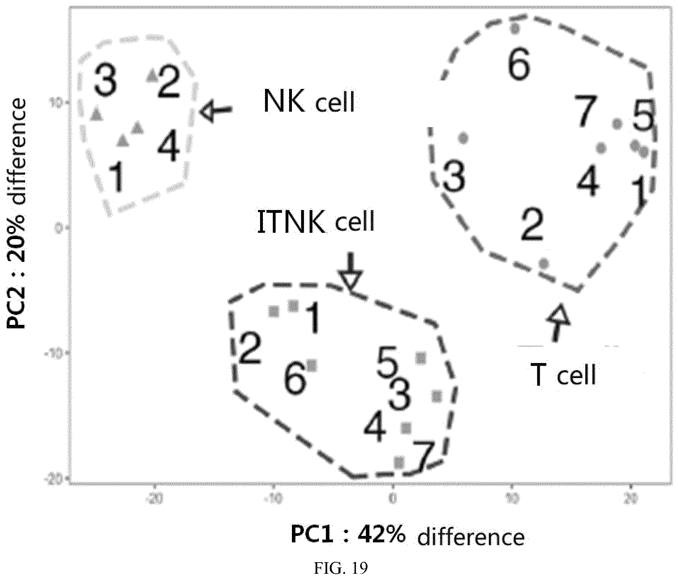

FIG. 19 shows the principal component analysis of RNA sequencing-cell subset gene expression data.

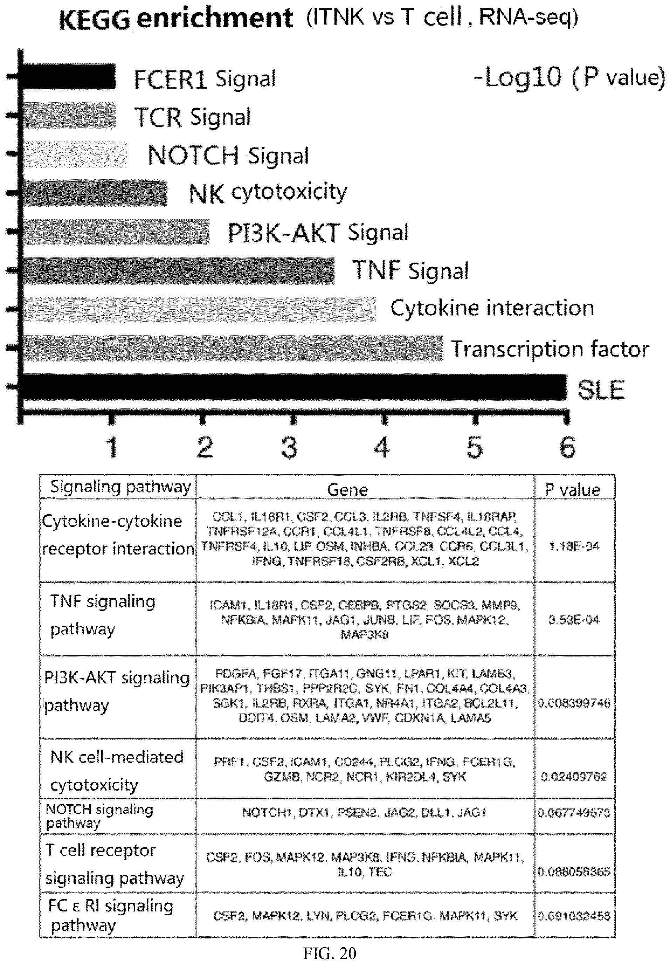

FIG. 20 shows RNA sequencing-KEGG enrichment pathway analysis of gene up-regulation of ITNK cells relative to T cells (cut-off: 2 times absolute logarithm, change ≥ 1; adjusted P value ≤ 0.05).

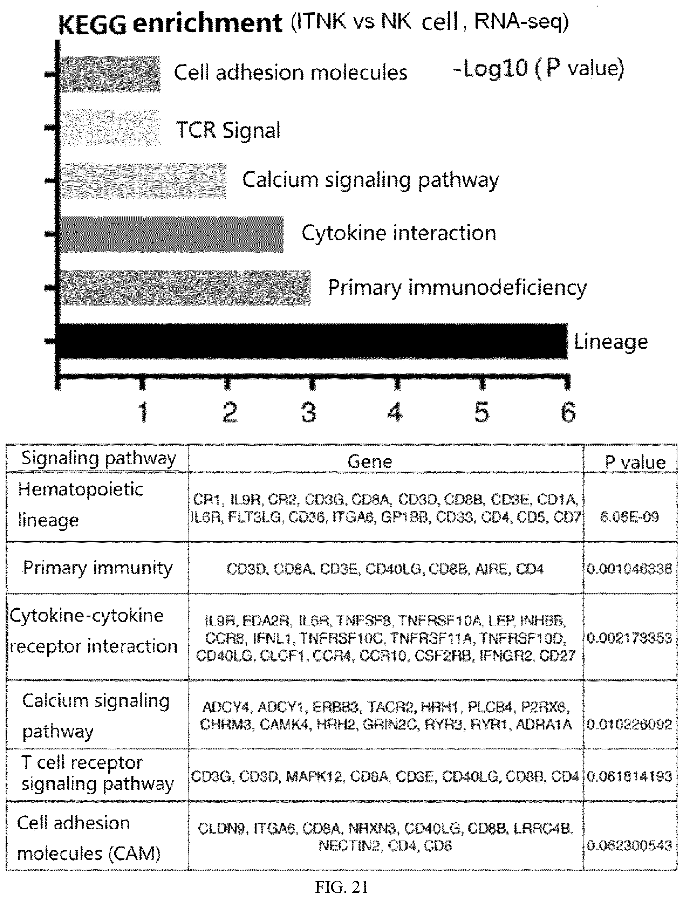

FIG. 21 shows RNA sequencing-KEGG enrichment pathway analysis of gene up-regulation of ITNK cells relative to NK cells (cut-off: 2 times absolute logarithm, change ≥ 1; adjusted P value ≤ 0.05).

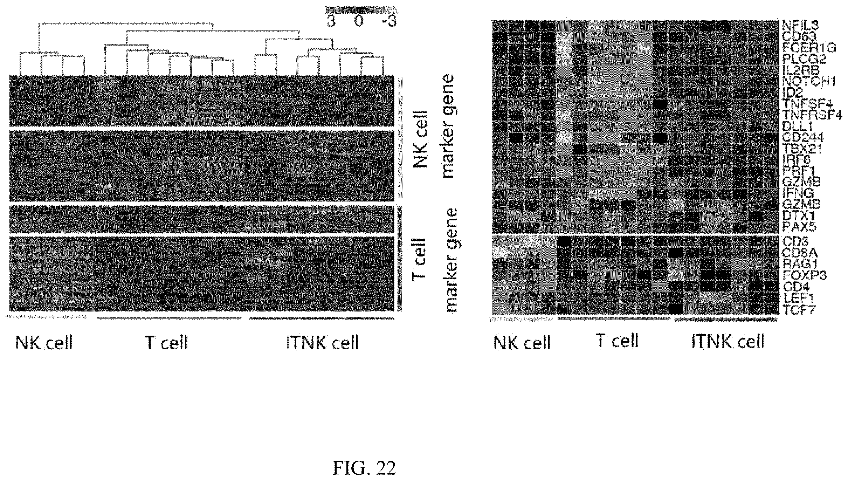

FIG. 22 is a hierarchical clustering heatmap of RNA-Seq, in which the left graph shows the difference of gene transcription expression among T cells, ITNK cells and NK cells (log2 absolute fold change ≥ 1; adjusted P value ≤ 0.05), and the right graph is heatmaps of differential expression of genes screened by RNA-seq analysis in T cells, ITNK cells and NK cells; and compared with T cells, the expression of NK signal gene in ITNK cells was up-regulated while the expression of TCF1 and LEF1 genes was down-regulated (log2 absolute fold change ≥ 1; adjusted P value ≤ 0.05).

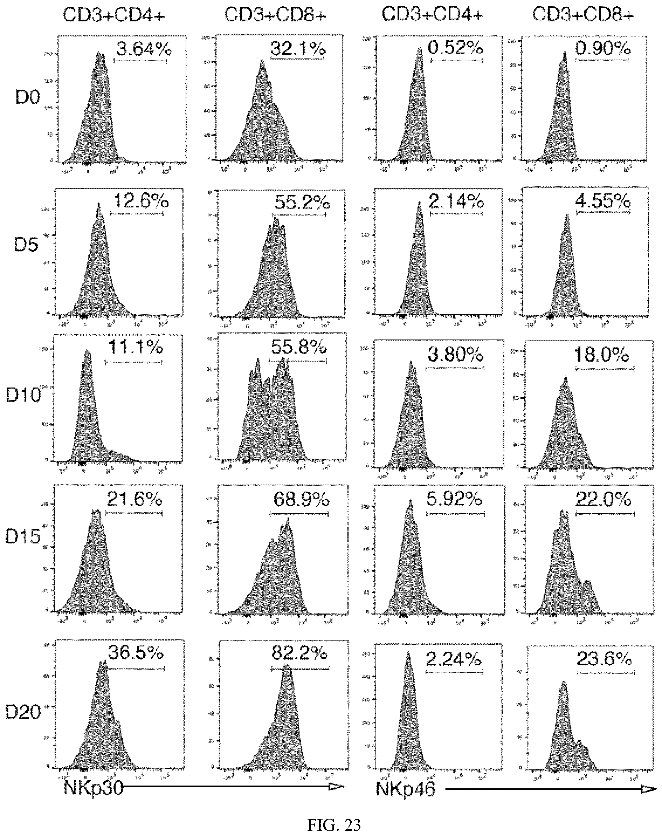

FIG. 23 is dynamic flow cytometric immunophenotype analysis images of ITNK cells; where the proportion of positive subsets NKp30 and NKp46 in subsets CD3+CD4+, CD3+CD8+ were detected by flow cytometry on day 0, day 5, day 10, day 15 and day 20 after BCL11B knockout in human T cells; the data represents three experiments; and NKp46 was detected on day 5 after BCL11B knockout and stabilized on day 10 to day 15.

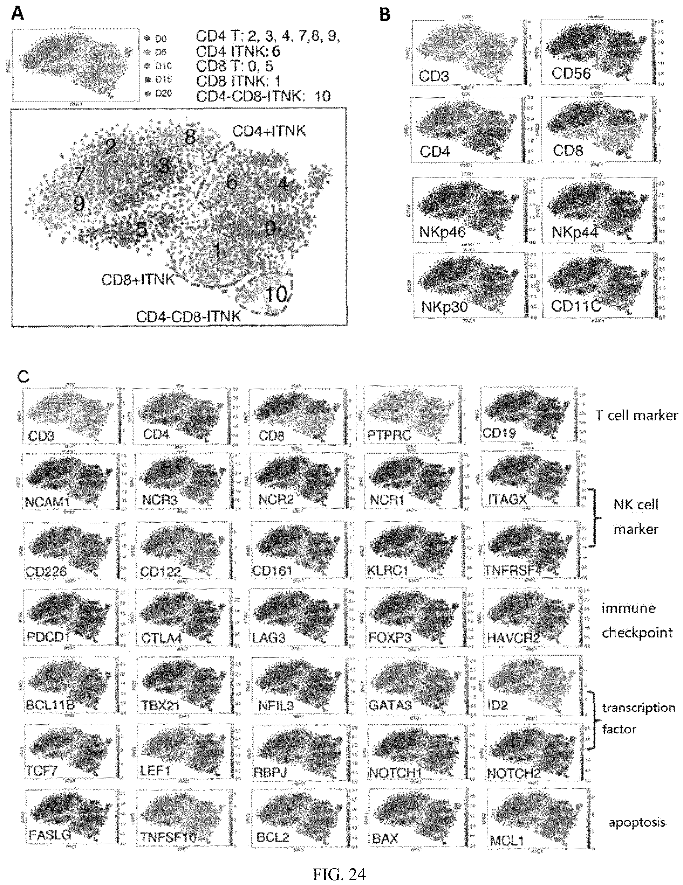

FIG. 24 (A) is a t-SNE dot plot of Sc-RNA seq analysis results, in which the upper graph shows the cell distribution of D0-D20, and the lower graph shows the clustering distribution of 4948 cells on Day D0 (2263), D5 (1565), D10 (498), D15 (204) and D20 (418) after PX458-gBCL11B gene-transduced T cells were subjected to BCL11B knockout, _{>}where 4948 cells were clustered into 11 cell subsets, and ITNK cells were mainly concentrated in subsets labeled by (red) circles; FIG. 24 (B) shows the expression of NK cell markers in 4948 detected cells (11 subsets) through t-SNE dot plots; and FIG. (C) shows the expression of T cell markers, NK cell markers, immune checkpoints, transcription factors and apoptosis gene-related genes in 4948 cells (11 subsets) through t-SNE dot plots.

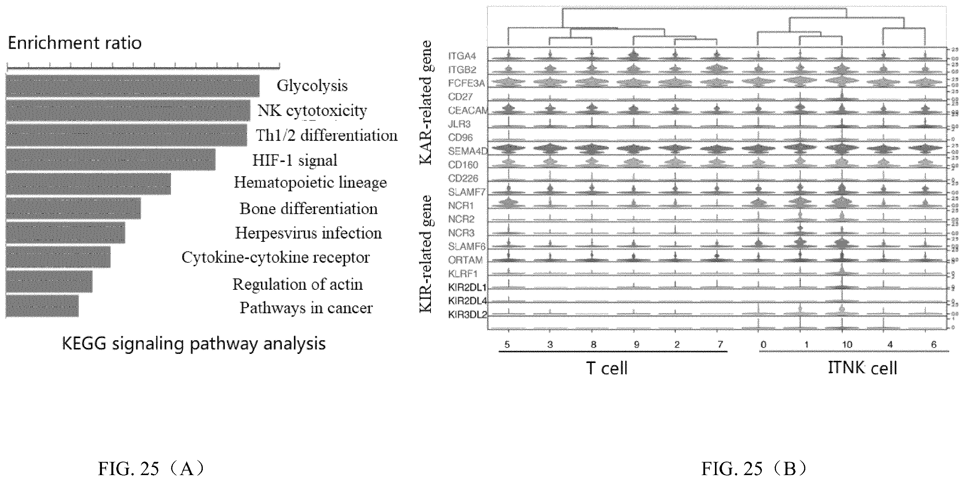

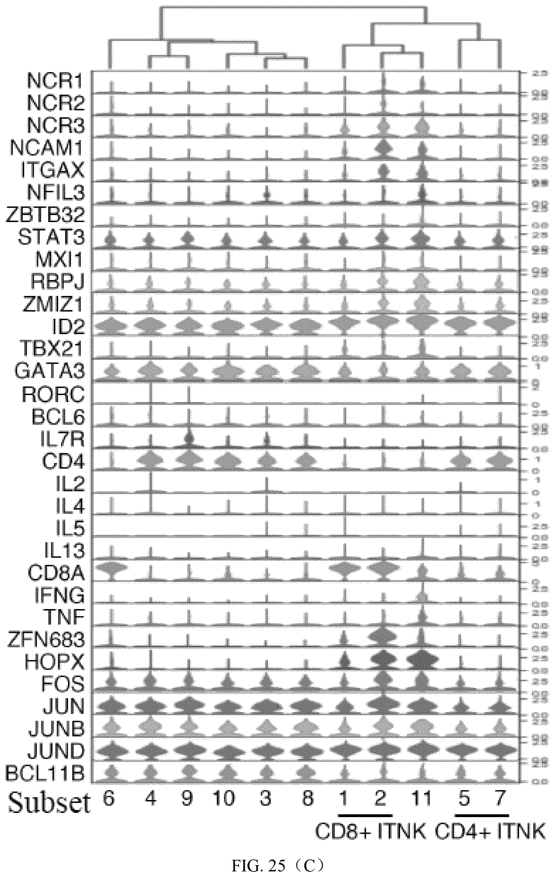

FIG. 25 (A) is KEGG signaling pathway analysis, showing high expression of NK cytotoxic genes in ITNK cells; FIG. 25 (B) is a violin plot, showing the expression profiles of NK cells-related KAR gene and NK cells-related KIR gene in different subsets; and FIG. 25 (C) is a violin plot, showing the expression profiles of genes related to T cell and NK cell development, where the expression of NK signal genes (ID2 and TBX21), NOTCH-related genes (MXI1, ZMIZ1, and RBPJ) and AP-1-related genes (FOS, JUN, JUNB, and JUND) was up-regulated in ITNK cells.

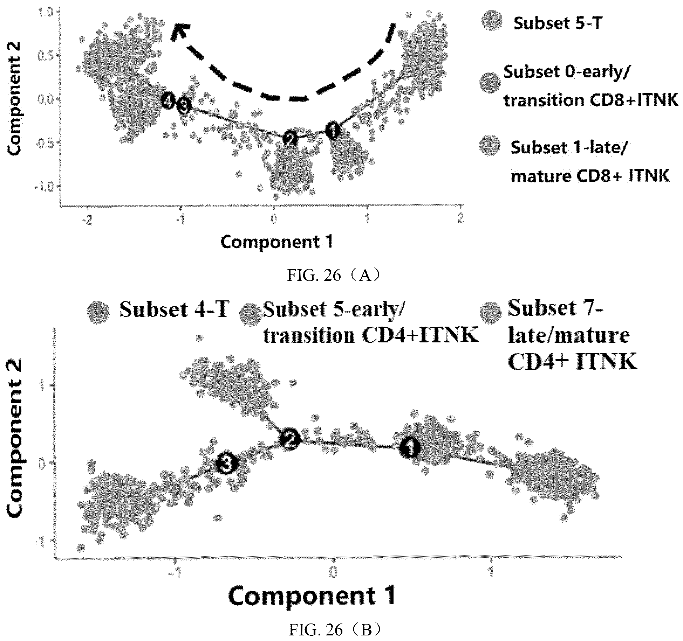

FIG. 26 (A) is an unsupervised trajectory analysis of individual cells from subset 5 (CD8+ T), subset 0 (early CD8+ ITNK) and subset 1 (late CD8+ ITNK), showing a gradual transition from CD8+ T cells to CD8+ ITNK cells; and FIG. 26 (B) is an unsupervised trajectory analysis of individual cells from subset 4 (CD4+ T), subset 5 (early CD4+ ITNK) and subset 7 (late CD4+ ITNK), showing a gradual transition from CD4+ T cells to CD4+ ITNK cells.

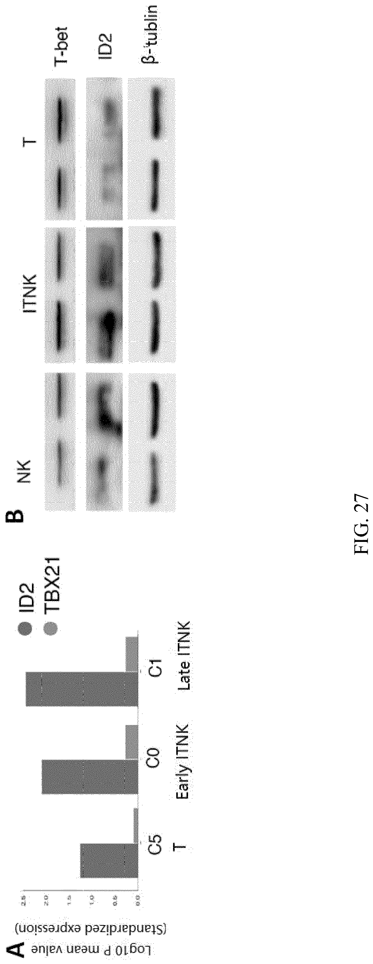

FIG. 27 (A) is a bar graph showing up-regulation of the expression of NK-related transcription factors ID2 and TBX21 in ITNK cells; and FIG. (B) is a Western Blot analysis of TBX21 and ID2 in immune cell lysate, in which NK cells were in the left lane, ITNK cells were in the middle lane, T cells were in the right lane, and β-Tublin was used as the internal reference.

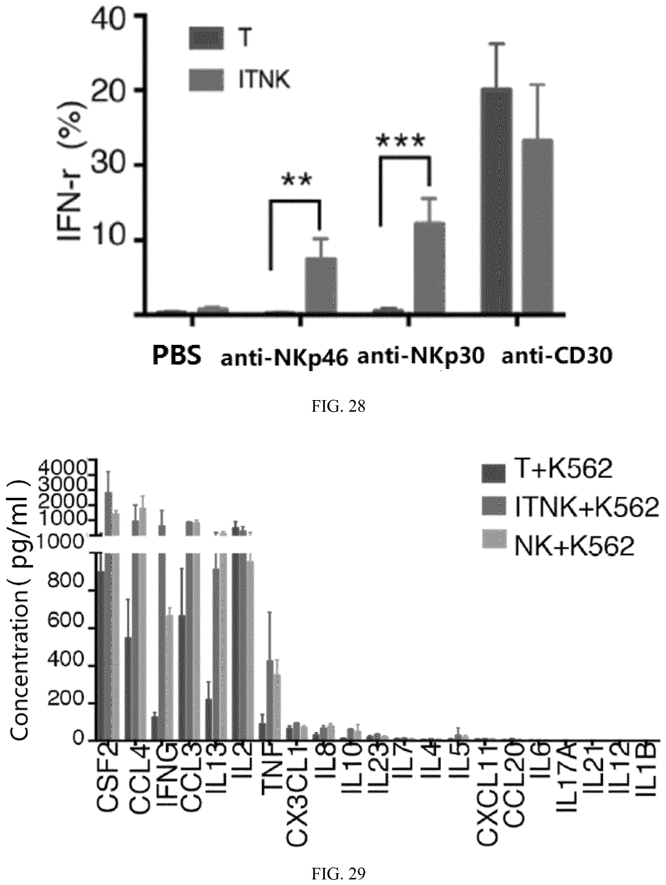

FIG. 28 is a histogram of the IFNγ secretion of T cells and ITNK cells after stimulated by anti-NKp30 antibody, anti-NKp46 antibody and anti-CD3/CD28 antibody (5 ug/ml), respectively, by Elisa assay, in which the data came from samples of three independent donors; the data were represented as mean±SD; ^{∗∗}P ≤ 0.01, and ^{∗∗∗}P ≤ 0.001; and a paired t test was adopted.
FIG. 29 shows the cytokine secretion of T cells, ITNK cells and NK cells after stimulated by K562 cells, where specifically, T cells, ITNK cells and NK cells were incubated with K562 cells at 37°C for 18 hours at an effector (E)-target (T) ratio of 1:1, respectively, the supernatant was collected for measuring the concentrations of cytokines (CSF2, CCL4, IFN γ, CCL3, IL13, IL2, TNF, CX3CL1, IL8, IL10, IL23, IL7, IL4, IL5, CXCL11, CCL20, IL6, IL17A, IL21, IL12, and IL1 β) by multiplex immunoassay; in which the value was expressed as the mean of three different donors.

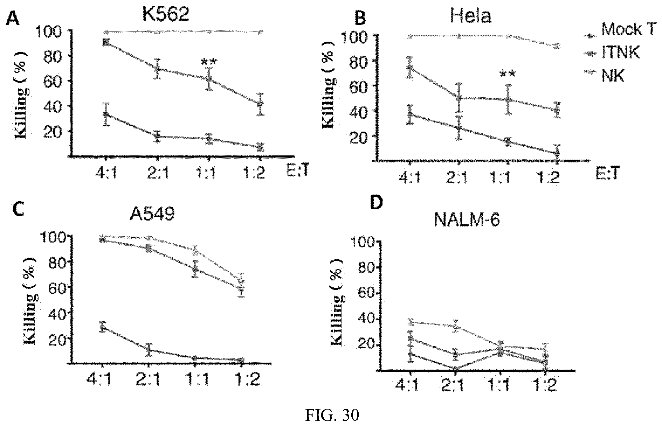

FIG. 30 shows the specific cytotoxicity percentages of ITNK cells to HLA-negative K562 cells (A), HLA-positive Hela cells (B), HLA-positive A549 cells (C), and HLA-positive NALM-6 cells (D), in which the data were expressed as mean±standard deviation (SD); ^{∗∗}P 0.01 0.01; an unpaired t-test was adopted.

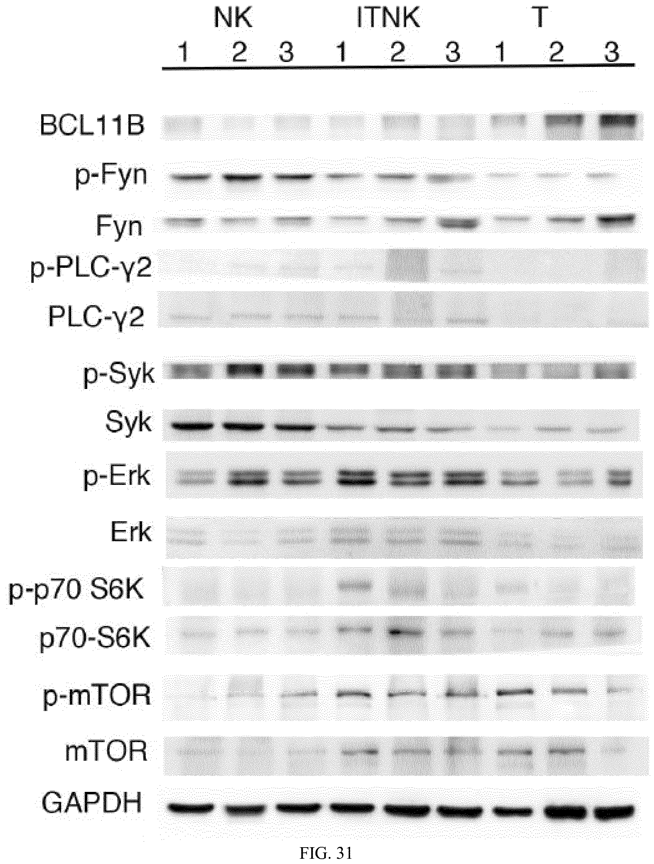

FIG. 31 shows the protein and phosphorylation levels of Fyn, PLC-g2, Syk, Erk1/2 and mTOR in immune cell lysate, measured by western blot after K562 cells were stimulated for 6 hours, in which NK cells were in the left three lanes, ITNK cells were in the middle three lanes, T cells were in the right three lanes, BCL11B was used as gene editing control, and GAPDH was used as sample control.

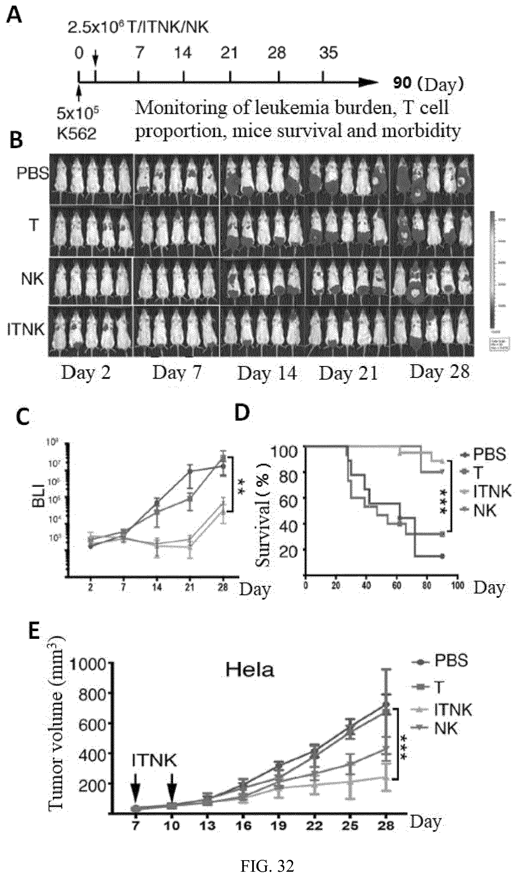

FIG. 32 (A) is a schematic diagram of the test for detecting the killing of tumor cells by ITNK cells *in vivo*; FIG. 32 (B) and (C) show the quantitative analysis of the total flux of luciferase activity *in vivo* in tested mice at specific time points by bioluminescence imaging (5 mice in each group), in which the results were mean±SD, ^{∗∗}P≤0.01, and an unpaired t test was adopted;
FIG. 32 (D) is a plot of survival time of K562 tumor-bearing mice treated with PBS (n=10), Mock T cells (n=15), ITNK cells (n=15) and NK cells (n=5); and FIG. 32 (E) shows the size of tumors in Hela tumor-bearing mice that were treated on day 7 and day 10 with PBS, Mock T cells, ITNK cells and NK cells respectively, measured at designated time points (5 mice in each group), in which the data were expressed as mean±standard deviation,^{∗∗∗}P≤0.001, and an unpaired t test was adopted.

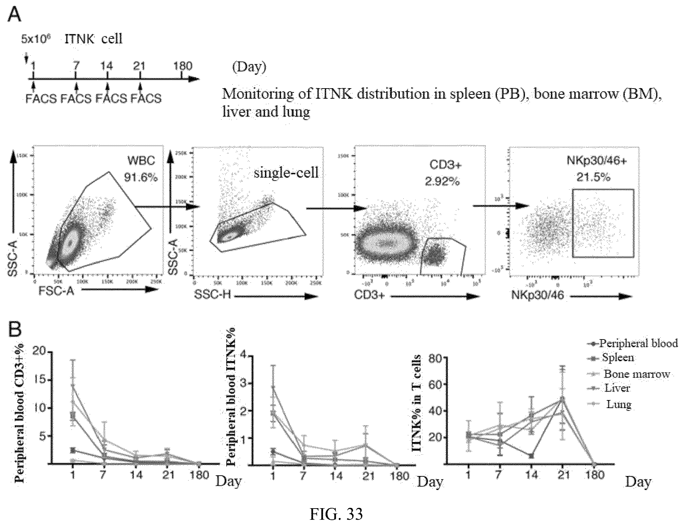

FIG. 33 shows the distribution and maintenance of ITNK cells in mice after ITNK cells were transplanted into NSI-strain immunodeficient mice lacking T cells, B cells and NK cells, in which, the top graph of (A) is a schematic diagram of injection of ITNK cells into NSI-strain mice and detection experiment, showing that ITNK cells were subjected to short-term analysis and long-term analysis on day 1, day 7, day 14, day 21 and month 6, respectively (n=3 in each group); the lower graph of (A) shows the percentage of ITNK cells in CD3+ T cells, analyzed by representative flow cytometry; and (B) shows the dynamic distribution of ITNK cells in peripheral blood (PB), bone marrow (BM), spleen, liver and lung of mice, showing that T cells and ITNK cells were not detected 6 months after the injection of ITNK cells.

### DETAILED DESCRIPTION

To further elaborate on the technical means adopted and the effects achieved in the present application, the solutions of the present application are further described below through specific examples in conjunction with drawings, but the present application is not limited to the scope of the examples.

The present application is not limited to the relative arrangement, numeric expressions and numerical values of the components and steps set forth in these examples, unless otherwise noted. Techniques, methods and devices known to those of ordinary skill in the art may not be discussed in detail, but in appropriate circumstances, techniques, methods and devices should be regarded as part of the specification.

### Example 1 Preparation of reprogrammed natural killer (ITNK) cells of the present application

### Construction of gene knockout plasmid vector

According to the selection rule of CRISP/CAS9 target sites: GN19NGG, where GN19 was a target site, N was still better G, and the target site can be on the antisense strand (i.e., the sequence order on the sense strand is CCN N19C), the following target sequences were selected and forward (F) and reverse (R) primers were designed as guideRNA (gRNA), respectively. The gRNA was annealed and ligated into the digested PX458 vectors to construct PX458-gBCL11B vectors (as shown in FIG. 1). PX458-gBCL11B was transfected in the 293 T cell line, and monoclones were selected. The knockout conditions such as base deletion and dislocation of BCL11B knockout targets were detected by gene sequencing (see FIG. 2), and the knockout efficiency was statistically calculated, as shown in Table 1 below.

**Table 1 BCL11B target sequences and their corresponding gRNAs list**

| Location | Target sequences | Forward and reverse primers | Specificity | Knockout efficiency |
|---|---|---|---|---|
| Second exon | GACCCTGACCTGC TCACCTG (SEQ ID NO: 1 ) | F: caccGACCCTGACCTGCTCACCTG (SEQ ID NO: 2) | 58 | 39 |
| | | R: aaacCAGGTGAGCAGGTCAGGGTC (SEQ ID NO: 3 ) | | |
| Second exon | GAAGCAGTGTGG CGGCAGCT (SEQ ID NO: 4) | F: caccGAAGCAGTGTGGCGGCAGCT (SEQ ID NO: 5 ) | 67 | 48 |
| | | R: aaacAGCTGCCGCCACACTGCTTC (SEQ ID NO: 6) | | |
| Second exon | CAGGTGGTCATCT TCGTCGG (SEQ ID NO: 7) | F: caccCAGGTGGTCATCTTCGTCGG (SEQ ID NO: 8) | 97 | 90 |
| | | R: aaacCCGACGAAGATGACCACCTG (SEQ ID NO: 9 ) | | |
| Second exon | GCAGGTGGTCATC TTCGTC (SEQ ID NO: 10) | F: caccGCAGGTGGTCATCTTCGTC (SEQ ID NO: 11 ) | 95 | 60 |
| | | R: aaacCGACGAAGATGACCACCTG (SEQ ID NO: 12) | | |
| Second exon | GCTCAGGAAAGT GTCCGAGC (SEQ ID NO: 13) | F: caccGCTCAGGAAAGTGTCCGAGC (SEQ ID NO: 14) | 86 | 59 |
| | | R: aaacGCTCGGACACTTTCCTGAG (SEQ ID NO: 15) | | |
| Second exon | GAGTCCCGTCACC CGAGACC (SEQ ID NO: 16) | F: caccGAGTCCCGTCACCCGAGACC (SEQ ID NO: 17) | 93 | 49 |
| | | R: aaacGGTCTCGGGTGACGGGACT (SEQ ID NO:18 ) | | |
| Third exon | GAAGTGATCACG GATGAGTG (SEQ ID NO: 19) | F: caccGAAGTGATCACGGATGAGTG (SEQ ID NO: 20) | 81 | 55 |
| | | R: aaacCACTCATCCGTGATCACTT (SEQ ID NO: 21) | | |
| Third exon | GGTGACGGGACTC AGGGTGA (SEQ ID NO: 22) | F: caccGGTGACGGGACTCAGGGTGA (SEQ ID NO: 23) | 62 | 69 |
| | | R: aaacTCACCCTGAGTCCCGTCAC (SEQ ID NO: 24) | | |
| Third exon | TGCAGCGCGCGCC CGGTCTC (SEQ ID NO: 25) | F: caccTGCAGCGCGCGCCCGGTCTC (SEQ ID NO: 26) | 87 | 63 |
| | | R: aaacGAGACCGGGCGCGCGCTGCA (SEQ ID NO: 27) | | |
| Fourth exon | CACGAGAGCGAC CCGTCGCT (SEQ ID NO: 28) | F: caccCACGAGAGCGACCCGTCGCT (SEQ ID NO: 29) | 99 | 49 |
| | | R: aaacAGCGACGGGTCGCTCTCGTG (SEQ ID NO: 30) | | |
| Fourth exon | GCGACGGGTCGCT CTCGTGG (SEQ ID NO:31) | F: caccGCGACGGGTCGCTCTCGTGG (SEQ ID NO: 32) | 97 | 69 |
| | | R: aaacCCACGAGAGCGACCCGTCG (SEQ ID NO: 33) | | |
| Fourth exon | TCCATGCTGAAGC TCGACTC (SEQ ID NO: 34) | F: caccTCCA TGCTGAAGCTCGACTC (SEQ ID NO: 35) | 91 | 60 |
| | | R: aaacGAGTCGAGCTTCAGCATGGA (SEQ ID NO: 36) | | |
| Fourth exon | ACGGGTCGCTCTC GTGGTGG (SEQ ID NO: 37) | F: caccACGGGTCGCTCTCGTGGTGG (SEQ ID NO: 38) | 90 | 92 |
| | | R: aaacCCACCACGAGAGCGACCCGT (SEQ ID NO: 39) | | |
| Fourth exon | AGCCGCAACCGC GAGAACGG (SEQ ID NO: 40) | F: caccAGCCGCAACCGCGAGAACGG (SEQ ID NO: 41) | 98 | 55 |
| | | R: aaacCCGTTCTCGCGGTTGCGGCT (SEQ ID NO: 42) | | |
| Fourth exon | GCAACTTGACGGT GCACCGG (SEQ ID NO: 43) | F: caccGCAACTTGACGGTGCACCGG (SEQ ID NO: 44) | 97 | 73 |
| | | R: aaacCCGGTGCACCGTCAAGTTG (SEQ ID NO: 45) | | |
| Fourth exon | GAGCTGGGCCGCC CGGGGCC (SEQ ID NO: 46) | F: caccGAGCTGGGCCGCCCGGGGCC (SEQ ID NO: 47) | 39 | 76 |
| | | R: aaacGGCCCCGGGCGGCCCAGCT (SEQ ID NO: 48) | | |
| Third exon | GGTCAGACGGAG GCTCCCTT (SEQ ID NO: 49) | F: caccGGTCAGACGGAGGCTCCCTT (SEQ ID NO: 50) | 61 | 65 |
| | | R: aaacAAGGGAGCCTCCGTCTGACC (SEQ ID NO: 51) | | |

According to the knockout efficiency in Table 1, the gRNA gene knockout plasmid vectors at the second exon and the third exon were selected to be knocked out for the next experiment. In the present application, preferably, BCL11B gene knockout was performed in the second exon and the third exon, the third pair of gRNA with the highest knockout efficiency was knocked out by using a mixture of the first pair of gRNA with the lowest knockout efficiency and the second pair of gRNA with the lowest knockout efficiency at the second exon, and the gene knockout plasmids corresponding to the third pair of gRNA with the lowest knockout efficiency at the third exon and the mixture thereof could reprogram T cells to obtain the immune killing lymphocytes of the present application. In this example, BCL11B gene knockout plasmids were constructed by using gRNA of SEQ ID NO: 5, SEQ ID NO: 6, SEQ ID NO: 50 and SEQ ID NO: 51, respectively, and mixed for the next experiment.

### Sorting and Activation of T Cells

T Cells were sorted and activated using the following method.
(1) Peripheral blood including human mature T cells and cord blood including human mature T cells were centrifuged at 300×g for 10 minutes, and plasma was collected and thermally inactivated at 56°C for 30 minutes.
(2) The precipitated granular blood cells were suspended with 0.9% NaCl, and peripheral blood mononuclear cells (PBMC) were separated by Ficoll density gradient centrifugation.
(3) Negative sorting was performed with MACS Pan T Cell Isolation kit (produced by Miltenyi Biotec in Bergisch Gladbach, Germany) to enrich all T cells (Pan T) from the blood such as peripheral blood and cord blood.

The above (1) to (3) are the steps of isolating human mature T cells from peripheral blood and cord blood, and it should be noted that other T cell sources are also acceptable, such as the directed differentiation of pluripotent stem cells and hematopoietic stem cells. T cells from all sources were activated with a T cell activation kit (produced by Miltenyi Biotec). Magnetic beads coated with anti-human CD3, anti-human CD28 antibody and anti-human CD2 were mixed and incubated with T cells in a ratio of 1:2 (cell density: 2.5×10⁶ cells/ml, and the medium: T551-H3 medium (produced by Takara, Japan) containing 5% autologous plasma, hIL2 (100 IU/ml), gentamicin sulfate (20 µg/ml), 10 mm of HEPES, 2 mm of glutamine and 1% penicillin/streptomycin) and then activated. After activation for 24 hours to 48 hours, T cells were eluted from antibiotin MACS iBead TM granules for later use.

### Induced reprogramming

(1) CRISP/CAS9 knockout vectors PX458-gBCL11B were transduced into the above-mentioned activated T cells by electrotransfer (T-023, LONZA Amaxa Nucleofector, Lonza).
(2) After 12 hours, T cells transduced with PX458-gBCL11B (such cells were referred to PX458-T) were centrifuged and cultured with T551-H3 (produced by Takara, Japan) medium (containing 5% autologous plasma or fetal bovine serum (FBS), 500 IU/ml hIL2 and gentamicin sulfate (20 µg/ml)).
(3) The fresh medium was changed every 3 days, and the cell density was kept in the range of 0.5×10⁶ cells/ml to 1×10⁶ cells/ml until day14 after electrotransfer.
(4) Whether the second exon and the third exon of BCL11B of T cells transduced with PX458-gBCL11B were subjected to knockout such as site induced insertion or deletion, was detected and verified by gene sequencing. The control group was T cells transduced with PX458 empty vectors (Mock).
(5) The expression level of BCL11B proteins in T cells transduced with PX458-gBCL11B was detected and verified by Western Blotting to further confirm the deletion of BCL11B proteins, and the control group was T cells transduced with PX458 empty vectors (Mock). The results of Western Blotting are shown in FIG. 3.

### Phenotype identification of reprogrammed cells

As described above, after 14 days of electrotransfer of T cells, 19.5% to 68.7% of the resulting cells expressed both T cell markers such as CD3 and NK cell markers such as NKp46, CD56, NKp30 and NKp44, and thus it was determined that the human ITNK cells of the present application were obtained. NK cells only expressed NK cell markers such as NKp46 and CD56, but did not express T cell markers such as CD3. T cells subjected to electrotransfer of empty vectors expressed T cell markers such as CD3, but did not express NK cell markers. The expression of cell markers of T cells, NK cells and ITNK cells is shown in FIGS. 4 to 6, and the phenotypic differences of these cells are also summarized in Table 2 below.

**Table 2 Phenotypic differences of ITNK cells, T cells and NK cells**

| -- | T cells | NK cells | ITNK cells |
|---|---|---|---|
| T cell marker | CD3, etc., | - | CD3, etc., |
| NK cell marker | - | High expression of NKp46, CD56, NKp30 and NKp44 | High expression of NKp46 (which is of no expression/low expression in CD4-positive ITNK cells), CD56, NKp30 and NKp44 |
| BCL11B expression | High expression | Low expression | Deletion |

In addition, according to the observation from the confocal microscope, the cell morphology of ITNK cells reprogrammed from T cells was different from the cell morphology of T cells and similar to the cell morphology of NK cells, and the reprogrammed ITNK cells had small nucleus (the volume of T cell nucleus relative to the whole cell), plentiful intracellular matrix, large granules, abundant endoplasmic reticulum and high protein synthesis activity, indicating that reprogrammed ITNK cells were immune killer lymphocytes. The transmission electron microscopic images of T cells, NK cells and ITNK cells are shown in FIG. 7.

In addition, the inventors also compared the expression profiles of these NK markers in the subsets of BCL11B-deficient T cells derived from cord blood and peripheral blood, and found that the percentages of CD8+NKP46+ cells and CD8+CD56+ cells were significantly higher than the percentages of CD4+NKP46+ cells and CD4+CD56+ cells, indicating that NKP46+CD3+ ITNK cells mainly derived from CD8+ T cells (see FIG. 8). Unlike CD8+ T cells, after the deletion of BCL11B, CD4+ T cells expressed NKp30 but didn't express NKp46 (see FIG. 8B).

CD4-CD8-NKp46+ subset expressed "TCRγδ" and was δγTCR+ ITNK cells (see FIG. 9). The deletion of BCL11B in CD4+ T cells, CD8+ T cells and δγTCR+ T cells was further verified by DNA sequencing (see FIG. 9).

### Example 2 Source identification of ITNK cells of the present application

TCRαβ sequencing: T cells derived from the same donor and ITNK cells obtained in Example 1 were subjected to RNA extraction and CDR3 region targeted amplification through human TCRαβ analysis kit to obtain TCR RNA. TCR RNA was subjected to sequencing on Hiseq 4000 platform to obtain a TCR library. Clustering combination analysis was performed with MiXCR(ref). The type of TCRαβ clone was derived with the parameter of "-chain" through the MiXCR clone derivation instruction. The diversity of TCR clones of T cells derived from the same donor and ITNK cells was compared by TCR sequencing, and it was found that the diversity of TCR clones was consistent (see FIGS. 10 and 11). Therefore, it was determined that the resulting ITNK cells were obtained by reprogramming of T cells with BCL11B deletion and maintained the TCR diversity of T cells, instead of being amplified from special and unknown small subsets of human T cells.

### Example 3 Single-cell immunophenotype identification of ITNK cells of the present application

ITNK cells obtained in Example 1 were subjected to single-cell immunophenotype analysis by mass cytometry (CyTOF) respectively, and the control group was T cells transduced with empty vectors.

Preparation and pretreatment of mass spectrometer samples: Cells from culture suspension were centrifuged, re-suspended with PBS containing 0.5% BSA and 0.02% NaN₃, and incubated with anti-human CD16/32 monoclonal antibody at room temperature for 10 minutes to block the Fc receptor. Then, a mixture of metal-labeled antibodies against cell surface molecules was added and incubated on ice for further 20 minutes. The antibodies were pre-coupled antibodies (produced by Fluidigm) or were internally coupled using a mass spectrometry flow coupling kit (produced by Fluidigm) according to the instructions. 5 mM of cisplatin was added to the cells, and the cells were incubated and stained on ice in FBS (produced by Fluidigm) for 1 minute. After the cells were treated with a fixation/permeabilization buffer (produced by Thermo Fisher), the cells were mixed and incubated with the metal-labeled antibodies to label intracellular proteins. After the cells were cleaned, the cells were stained with 1 mL of 191/193Ir DNA intercalator (produced by Fluidigm) that was diluted at a ratio of 1:4000 (the intercalator was diluted with PBS containing 1.6% paraformaldehyde (produced by EMS)) and then stored at 4°C. Before detection, the cells were washed once with PBS containing 0.5% BSA and 0.02% NaN₃ and once with ddH₂O, then re-suspended and diluted to about 10⁶ cells/ml with ultrapure water (ddH₂O). Subsequently, cell sample data were detected and collected using CyTOF2 device (produced by Fluidigm) at an event rate of <400 events/sec.

According to the cellular immunophenotypic differences of 40 markers, clustering analysis was performed through PhenoGraph clustering algorithm. ITNK cells derived from cord blood (hereinafter referred to as CB-ITNK), ITNK cells derived from peripheral blood (hereinafter referred to as PBMC-ITNK) and Mock-T cells were integrated and classified into 39 subsets, as shown in FIGS. 12, 13, 14, 15, 16 and 17. As can be seen from FIG. 12, PX458-T cells and Mock-T cells were separated before and after stimulation by K562 cells, and there was almost no overlap, indicating that there was a significant difference between PX458-T cells and Mock-T cells; there was obvious overlap between PX458-T cells before and after stimulation by K562 cells, and more new subsets were derived from activated PX458-T cells.

According to results of cell marker expression heterogeneity analysis by mass cytometry, the ITNK cells of the present application included CD3-negative cell subset of NO. 33, CD4+ cell subsets of NOs. 5 to 10, CD8+ cell subsets of NOs. 20 to 22 and 26 to 28, and TCRγδ+ cell subsets of NOs. 23 to 24, and all expressed NK-related markers such as CD56, NKp30, NKp44, NKp46 or CD11C, and compared with γδT cells, NKp46, NKp30 and NKp44 markers were of high expression in TCRyδ+ ITNK cells, i.e., (NKp46^{high} NKp30^{high} NKp44^{high}) (as shown in FIGS. 13 and 14A). The ITNK cells of the present application differed from conventional NK cells in that the ITNK cells of the present application didn't express CD127, CD16, NKG2A and KIR2DL2 (see FIG. 14B). Moreover, immunosuppression checkpoints PD-1, CTLA-4 and FOXP3 were of low expression in the ITNK cells of the present application (as shown in FIG.14C), and since the high expression of immunosuppression checkpoints will induce immune cells to form immunosuppressive states such as low function and failure, it is indicated that the ITNK cells of the present application had a strong immune effect and were not easily suppressed by immunosuppressive microenvironments such as tumors.

In addition, in ITNK cells derived from cord blood, the histogram as shown in FIG. 15 clearly shows the percentages of various ITNK cells in CD45+ hematopoietic cells and the dynamic transition from static ITNK cells to effector cells after stimulation. As shown in FIG. 16, the dynamic transition of ITNK cells derived from adult peripheral blood from resting state to effector state after stimulation was the same as the dynamic transition of ITNK cells derived from cord blood. As shown in FIG. 17, the mass cytometry heatmap shows the dynamic changes of immunophenotype of ITNK cells before and after activation, and the expression of CD25 increased after activation. To sum up, ITNK cells, after activated, still remain the expression of NK cell activation receptors and T cell markers.

### Example 4 RNA-Seq transcription profile analysis of ITNK cells of the present application

In order to study the entire gene expression profile of ITNK cells, the inventors performed RNA sequencing analysis on T cells derived from 4 cord blood samples and 3 adult peripheral blood samples, ITNK cells derived from 4 cord blood samples and 3 adult peripheral blood samples, and NK cells derived from 2 cord blood samples and 2 adult peripheral blood samples. The sorting operation is as follows: flow cytometry analysis or sorting was performed by flow cytometers Canto, FACS Fortessa (BD), FACSAriaII, etc. Cell-surface receptor labeling: the cells and antibody were mixed in 50 µl of flow buffer (PBS solution containing 2% FBS) and incubated at 4°C for 30 minutes in the dark. Cell intracellular labeling: the cells were subjected to permeable treatment with Foxp3/transcription factor staining buffer (produced by eBioscience), after the buffer was eluted, blocked with mouse serum or rabbit serum, incubated with antibodies at 4°C for 30 minutes in the dark, washed with flow buffer and then suspended for subsequent flow cytometry analysis or sorting. Cell sorting strategy and sorting purity were verified (as shown in FIG. 18).

Principal component analysis (PCA) was performed on RNA sequencing results of 18 samples for similarity evaluation, and it was found that ITNK cells were different from T cells and NK cells according to transcriptome analysis (as shown in FIG. 19). Compared with T cells, 776 genes were increased (data were not shown), including NK specific transcription factors (such as ID2, TBX21, NFIL3, and IRF8), NK cell activation and inhibition receptors/proteins (such as IL2RB, IFNG, PRF1, GZMB, TNFRSF4, NCR1, NCR2, and PLCG2), and histone genes (HIST1H1D, HIST1H2AC/D/E/G/M, and HIST2H2A3/4) (as shown in FIGS. 22 and 20). On the contrary, compared with T cells, 592 genes such as TCF-1/TCF-7, LEF-1, IL-7R, MYC, PD-L1, FOXP3, etc. were down-regulated in ITNK cells (as shown in Table 3). Compared with NK cells, the expression of 666 genes in ITNK cells increased (data were not shown), and most of these genes were enriched in T cell recognition and TCR signal transduction (CD3, CD4, CD8, CD40LG) (FIG. 21). Interestingly, compared with NK cells, the expression of KIR genes KIR2DL1, KIR2DL3, KIR3DL1 and KIR3DL2 in ITNK was down-regulated (as shown in Table 4), while KIR2DL1, KIR2DL3, KIR3DL1 and KIR3DL2 were NK cell inhibition receptors and mediated the inhibition of immune cells, and their low expression indicates that ITNK cells had resistance to immunosuppressive microenvironments. The results of whole transcriptome sequencing analysis show that the expression of NK cell marker genes IL2RB, ID2 and NFIL3 was up-regulated in ITNK cells (shown in the right image of FIG. 22).

**Table 3 Expression of related genes of ITNK cells relative to NK cells**

| ENTRE Z ID | Marke r | Basic value | log2 fold change | lfcSE | stat | P value | padj |
|---|---|---|---|---|---|---|---|
| 6932 | TCF7 | 6118.3958 8 | -1.705017 1 | 0.2063406 6 | -8.263117 6 | 1.4192E-16 | 3.4959E-14 |
| 51176 | LEF1 | 4011.1231 5 | -1.145179 1 | 0.1591786 6 | -7.194300 3 | 6.2782E-13 | 6.9436E-11 |
| 3575 | IL7R | 1810.0315 7 | -3.993120 8 | 0.4030516 9 | -9.907217 7 | 3.8728E-23 | 6.2964E-20 |
| 4609 | MYC | 1160.2289 7 | -2.686192 9 | 0.2856978 2 | -9.402216 7 | 5.3425E-21 | 4.1361E-18 |
| 50943 | FOXP 3 | 769.30224 6 | -2.021528 9 | 0.5842629 8 | -3.459964 | 0.000540 25 | 0.004150 92 |

**Table 4 Expression of related genes of ITNK cells relative to NK cells**

| ENTRE Z ID | Marker | Basic value | log2 fold change | lfcSE | stat | P value | padj |
|---|---|---|---|---|---|---|---|
| 3811 | KIR3D L1 | 365.0991 98 | -7.1254794 | 0.751468 44 | -9.48207 4 | 2.4928E-21 | 3.971E-1 8 |
| 3804 | KIR2D L3 | 473.4124 88 | -4.8525337 | 0.890729 48 | -5.44781 98 | 5.0991E-08 | 2.9645E-06 |
| 3812 | KIR3D L2 | 373.9606 1 | -3.0917182 | 0.861439 47 | -3.58901 39 | 0.000331 93 | 0.003785 01 |
| 3802 | KIR2D L1 | 282.0004 46 | -4.7696308 | 0.991475 86 | -4.81063 73 | 1.5045E-06 | 5.0993E-05 |

### Example 5 Single-cell transcriptome sequencing analysis of ITNK cells of the present application

Flow cytometry shows that CD8+CD3+NKp46+ ITNK cells and CD4+CD3+NKp30+ ITNK cells appeared on day 5 after BCL11B was knocked out (as shown in FIG. 23). In order to further explain the cell fate transition during reprogramming of T cells to ITNK cells, through micropore-based single-cell transcriptome sequencing (scRNA-seq), the gene expression profiles of T cells and ITNK cells in CD3+ cord blood samples at different time points after BCL11B knockout were studied.

About 5000 cells were detected and analyzed in all experimental groups. Different time-point groups of cell samples were detected by scRNA-seq, 2000 to 4000 genes per cell were detected, and a total of 20000 human genes were detected among all cells. In the t-distributed random neighbor-embedded (t-SNE) analysis of transcription profiles, the cells were projected into two dimensions, which provided a visual representation of the cell fate transition in the process of ITNK cell reprogramming. The results of the unbiased t-SEN analysis show that the cells from day 0 to day 20 after knockout could be clustered into 11 subsets (as shown in FIG. 24). According to the expression of marker genes in T cells and NK cells, it is determined that ITNK cells were mainly concentrated in subset 6 (CD4+ ITNK), subset 1 (CD8+ ITNK) and subset 10 (γδTCR+ ITNK) (as shown in FIG. 24), and these subsets completely coincided with the BCL11B-deleted subset, further indicating that ITNK cells were reprogrammed by knocking out BCL11B in T cells. KEGG enrichment analysis shows that NK marker genes and related genes were of specific high expression in ITNK cells (as shown in FIG. 25). As can be seen from FIGS. 24C and 25C, NOTCH1, NOTCH2, ZMIZ1 (NOTCH1 cofactor) and RBPJ (NOTCH downstream transcription factor) were up-regulated in human ITNK cells, indicating that the NOTCH signaling pathway played a role in reprogramming of ITNK cells. FOS, JUN, JUNB, the three AP-1 transcription factor subunits were of low expression in the early stage of ITNK reprogramming, but gradually up-regulated in the late stage of ITNK reprogramming (see FIG. 25C). As can be seen, the NOTCH signal and the AP-1 signal were up-regulated after ITNK reprogramming. T cells and ITNK cells were closely aggregated in the t-SNE dot plots, indicating that the transition from T cells to NK cells was almost synchronous. As can be seen from the analysis of the trajectory of NK marker genes of CD8+ T (subset 5), early CD8+ ITNK (subset 0) and late CD8+ ITNK (subset 1), there wasa gradual transition from CD8+ T cells to CD8+ ITNK cells (see FIG. 26A). Similarly, the analysis of the trajectory of NK marker genes of CD4+ T cells and ITNK cells also shows the gradual transition from T cells to ITNK cells (FIG. 26B). Consistent with the results of dynamic flow cytometric immunophenotype analysis of ITNK cells (FIG. 23), T cells began to reprogram into ITNK cells on day 5 after knockout of BCL11B. It is found that the expression of transcription factor genes (TBX2, ID2, etc.) was significantly up-regulated during the reprogramming of T cells to ITNK cells, which was further verified by western blotting (as shown in FIG. 27).

### Example 6 Ability of ITNK cells of the present application to recognize and kill MHCI-positive/negative tumor cells in vitro

In order to determine whether the NK cell receptor (NCR) and T cell receptor (TCR) expressed on ITNK cells of the present application are functional, the ITNK cells were stimulated with anti-NKp30 monoclonal antibody, anti-NKp46 monoclonal antibody and anti-CD3/CD28 monoclonal antibody, respectively. It is found that the secretion of interferon (IFN) in ITNK cells increased after the ITNK cells were stimulated with anti-NKp30 antibody and anti-NKp46 antibody, while the secretion of IFN in T cells of the control group did not increase (as shown in FIG. 28); and the secretion of interferon in ITNK cells increased after the ITNK cells were stimulated with anti-CD3/CD28 antibody, while the secretion of interferon in T cells of the control group did not increase (as shown in FIG. 28). It is indicated that the NCR and the TCR in ITNK cells were functional.

Similar to NK cells, ITNK cells of the present application could secrete a variety of cytokines, including GM-CSF, IFN, and TNF (as shown in FIG. 29), and recognize and kill MHC-I negative K562 cells (as shown in FIG. 30A). In addition, ITNK cells could effectively kill Hela cells and A549 cells (as shown in FIG. 30B and 30C), and the two cells are ligands with high expression of NK-activated receptors and are MHC I-positive; and as shown in Table 2, since NK cell KIR receptors (KIR2DL1, KIR2DL3, KIR3DL1 and KIR3DL2) which mediated immunosuppression were of low expression in ITNK cells, ITNK cells had a better tumor-killing effect than NK cells. However, NALM-6 did not express the NCR ligand and highly expressed MHC-I molecules, and ITNK cells and NK cells had no significant killing effect on NALM-6 (as shown in FIG. 30D). Then, the inventors stimulated ITNK cells, NK cells and T cells with K562 cells, respectively, and it is found that the expression levels of phosphorylated Fyn, PLC-y2, Syk, Erk and m-TOR in ITNK cells and NK cells were similar, but higher than the expression levels in T cells (as shown in FIG. 31). These results indicate that ITNK cells had similar NK cell functions in NCR activation, cytokine secretion, cytotoxicity and signaling pathway.

### Example 7 Ability of ITNK cells of the present application to inhibit tumor growth in vivo

The inventors also evaluated whether the ITNK cells of the present application could inhibit the growth of xenograft tumors. Specifically, K562 cells labeled with luciferase were implanted into NSI mice to construct K562 tumor-bearing mouse models, and then ITNK cells, NK cells or T cells were injected in a single time (FIG. 32A). The survival status of K562 cells in mice *in vivo* was detected at specific time points by living imaging equipment. Compared with the group injected with T cells (negative control) and the group injected with PBS (blank control), K562 tumor burden in the experimental mice treated with ITNK cells and NK cells significantly reduced after 28 days of injection (FIGS. 32B and 32C), and these mice survived longer (FIG. 32D). In addition, the inventors also transplanted Hela cells into NSI mice and treated Hela xenograft mice with ITNK cells, NK cells or T cells, respectively. The results show that the growth rate of Hela tumor in tumor-bearing mice treated with ITNK cells was significantly slower than the growth rate in the group treated with NK cells, the group treated with T cells or the group treated with PBS (FIG. 32E). As can be seen, the ITNK cells of the present application were effective killers of tumor cells *in vivo* and could prevent tumor progression.

### Example 8 In vivo safety assessment of ITNK cells of the present application

In order to verify the distribution and maintenance ability of ITNK cells *in vivo,* ITNK cells were transplanted into NSI-strain immunodeficient mice lacking T cells, B cells and NK cells, and the percentage of ITNK cells in peripheral blood (PB), spleen (SP), bone marrow (BM), liver, and lung was measured on day 1, day 7, day 14, day 21 and day 180 after transplantation (FIGS. 33A and 33B). The proportion of ITNK cells peaked on day 21 after transplantation, then gradually decreased, and could not be detected after 6 months (FIG. 33B). As can be seen from FIG. 33B, ITNK cells had better maintenance ability *in vivo* than T cells. The case where ITNK cells attack the host or amplified without restriction was not observed.

In order to evaluate the possible off-target mutation induced by PX458-gBCL11B, T cells electroporated with PX458-gBCL11B were subjected to the whole genome sequencing of high coverage. Compared with wild-type T cells, it is found from two independent experiments that there were very few off-target mutations caused by nuclease in T cells edited by PX458-gBCL11B.

The applicant has stated that although the detailed method of the present application is described through the examples described above, the present application is not limited to the detailed method described above, which means that implementation of the present application does not necessarily depend on the detailed method described above. It should be apparent to those skilled in the art that any improvements made to the present application, equivalent replacements of raw materials of the product of the present application, additions of adjuvant ingredients to the product of the present application, and selections of specific manners, etc., all fall within the protection scope and the disclosed scope of the present application.

## Claims

1. Immune killer lymphocytes induced by reprogramming of human T cells, which retain markers and functions of human T cells from which the immune killer lymphocytes are derived and have markers and functions of NK cells, wherein the reprogramming of the human T cells involves deletion of a BCL11B gene.

2. The immune killer lymphocytes according to claim 1, wherein the human T cells are mature human T cells or a cell population comprising mature human T cells;
preferably, the mature human T cells or the cell population comprising the mature human T cells are derived from human cord blood or human peripheral blood; and
preferably, the mature human T cells or the cell population comprising the mature human T cells are derived from mature T cells or cell populations obtained by differentiation of pluripotent stem cells, embryonic stem cells or cord blood stem cells.

3. The immune killer lymphocytes according to claim 1 or 2, expressing functional TCR, CD3 and NKp30.

4. The immune killer lymphocytes according to any one of claims 1 to 3, expressing a marker of NK cells selected from the following group consisting of CD11c, NKG2D and CD161;
preferably, immunosuppression checkpoints PD-1, CTLA-4 or FOXP3 are of low expression or no expression in the immune killer lymphocytes; and
preferably, NK-related markers CD127, CD16, KIRDL2, KIRDL3 and NKG2A are of low expression or no expression in the immune killer lymphocytes.

5. The novel immune killer lymphocytes according to any one of claims 1 to 4, wherein compared with the T cells from which the immune killer lymphocytes are derived, expression of NOTCH is up-regulated

6. The immune killer lymphocytes according to any one of claims 1 to 5, wherein compared with the T cells from which the immune killer lymphocytes are derived, expression of transcription factors LEF1 and TCF7 is down-regulated, and expression of NOTCH, API, ID2, TBX21 and NFIL3 is up-regulated.

7. The immune killer lymphocytes according to any one of claims 1 to 6, wherein TCR-mediated signal transduction of the immune killer lymphocytes is enhanced;
preferably, compared with the T cells from which the immune killer lymphocytes are derived, expression of genes of the cells, which are related to the TCR-mediated signal transduction and comprise CSF2, FOS, MAPK12, MAP3K8, IFNγ, NFKBIA, MAPK11, IL-10 and TEC, is up-regulated; and
preferably, compared with NK cells, T cell recognition and TCR signal transduction of the immune killer lymphocytes are enhanced; and preferably, expression of CD3, CD4, CD8 and CD40LG is up-regulated.

8. The immune killer lymphocytes according to any one of claims 1 to 7, wherein compared with the T cells from which the immune killer lymphocytes are derived, NK killing toxicity-related signal transduction of the immune killer lymphocytes is enhanced; and
preferably, compared with the T cells from which the immune killer lymphocytes are derived, expression of genes of the immune killer lymphocytes, which are related to the NK killing toxicity-related signal transduction and comprise PRF1, CSF2, ICAM1, CD244, PLCG2, IFNG, FCER1G, GZMB, NCR2, NCR1, KIR2DL4 and SYK, is up-regulated.

9. The immune killer lymphocytes according to any one of claims 1 to 8, comprising cell subsets CD8+NKp46+NKp44+NKp30+, CD4+NKp30+, and γδTCR+NKp46+NKp44+NKp30+T.

10. The immune killer lymphocytes according to any one of claims 1 to 9, wherein the human T cells are mature human T cells, and the reprogramming of the human T cells comprises:
1) activating mature human T cells;
2) performing BCL11B gene knockout on the activated mature human T cells obtained in step 1); and
3) culturing the cells obtained in step 2) with a T cell culture medium.

11. The immune killer lymphocytes according to claim 10, wherein in step 1), the activation is performed using an anti-human CD3 antibody, an anti-human CD28 antibody, and an anti-human CD2 antibody; and
preferably, magnetic beads of anti-human CD3 antibody, anti-human CD28 antibody and anti-human CD2 antibody are mixed with the mature human T cells in a ratio of 1:2 and incubated to activate the T cells.

12. The immune killer lymphocytes according to claim 10 or 11, wherein in step 2), the BCL11B gene knockout is performed by CRISPR/CAS9;
preferably, a target of the gene knockout is at a second exon of the BCL11B gene; and
preferably, the target of the gene knockout is at a third exon of the BCL11B gene.

13. The immune killer lymphocytes according to any one of claims 10 to 12, wherein in step 3), the T cell medium comprises IL-2; and preferably, the cells obtained in step 2) are not co-cultured with OP9-DL1.

14. A method for preparing the immune killer lymphocytes according to any one of claims 1 to 13, comprising:
1') activating human T cells;
2') performing BCL11B gene knockout on the activated human T cells obtained in step 1'); and
3') culturing the cells obtained in step 2') with a T cell culture medium.

15. The method according to claim 14, wherein the human T cells are mature human T cells or a cell population comprising mature human T cells;
preferably, the mature human T cells or the cell population comprising the mature human T cells are derived from human cord blood or human peripheral blood; and
preferably, the mature human T cells or the cell population comprising the mature human T cells are derived from mature T cells or cell populations obtained by differentiation of pluripotent stem cells, embryonic stem cells or cord blood stem cells.

16. The method according to claim 14 or 15, wherein in step 1'), the activation is performed using an anti-human CD3 antibody, an anti-human CD28 antibody, and an anti-human CD2 antibody; and
preferably, magnetic beads of anti-human CD3 antibody, anti-human CD28 antibody and anti-human CD2 antibody are mixed with mature human T cells in a ratio of 1:2 and incubated to activate the T cells.

17. The method according to any one of claims 14 to 16, wherein in step 2'), the BCL11B gene knockout is performed by CRISPR/CAS9;
preferably, the gene knockout is performed at a second exon of a BCL11B gene; and
preferably, the gene knockout is performed at a third exon of the BCL11B gene.

18. The method according to any one of claims 14 to 17, wherein in step 3'), the T cell medium comprises IL-2; and preferably, the cells obtained in step 2') are not co-cultured with OP9-DL1.

19. An application of the immune killer lymphocytes according to any one of claims 1 to 13 in the preparation of a medicament for the treatment of a disease selected from the group consisting of tumors, AIDS and infectious diseases; and preferably, the infectious diseases are viral infectious diseases.
